(19) **Europäisches Patentamt**
**European Patent Office**
**Office européen des brevets**

(11) **EP 4 601 737 B1**

(12) **EUROPEAN PATENT SPECIFICATION**

(45) Date of publication and mention
of the grant of the patent:
**01.07.2026 Bulletin 2026/27**

(21) Application number: **24804683.1**

(22) Date of filing: **24.10.2024**

(51) International Patent Classification (IPC):
***A61N 1/372*** (2006.01)

(52) Cooperative Patent Classification (CPC):
**A61N 1/3727; A61B 5/0028; A61B 5/0031;**
**A61N 1/37217; A61N 1/37252; A61N 1/37288;**
**H04B 13/005;** A61B 5/0006; A61B 5/0538;
A61B 5/29

(86) International application number:
**PCT/US2024/052867**

(87) International publication number:
**WO 2025/090803 (01.05.2025 Gazette 2025/18)**

(54) **IMPROVED COMMUNICATION BETWEEN MEDICAL DEVICES**

VERBESSERTE KOMMUNIKATION ZWISCHEN MEDIZINISCHEN VORRICHTUNGEN

AMÉLIORATION DE LA COMMUNICATION ENTRE DES DISPOSITIFS MÉDICAUX

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **26.10.2023 US 202363593323 P**

(43) Date of publication of application:
**20.08.2025 Bulletin 2025/34**

(60) Divisional application:
**26176835.2 / 4 768 072**

(73) Proprietor: **Pacesetter, Inc.**
**Sylmar, CA 91342 (US)**

(72) Inventors:
• **XIN, Xiyao**
**Sylmar, California 91342 (US)**
• **SISON, Shiloh**
**Sylmar, California 91342 (US)**
• **CHEN, Xi Lin**
**Sylmar, California 91342 (US)**

(74) Representative: **Barker Brettell Sweden AB**
**Kungsbroplan 3**
**112 27 Stockholm (SE)**

(56) References cited:
**US-A1- 2022 370 810**

• **ANNA VIZZIELLO ET AL: "Intra-Body**
**Communications for Nervous System**
**Applications: Current Technologies and Future**
**Directions", ARXIV.ORG, CORNELL UNIVERSITY**
**LIBRARY, 201 OLIN LIBRARY CORNELL**
**UNIVERSITY ITHACA, NY 14853, 3 April 2023**
**(2023-04-03), XP091483790, DOI: 10.1016/**
**J.COMNET.2023.109718**
• **DATABASE COMPENDEX [online]**
**ENGINEERING INFORMATION, INC., NEW YORK,**
**NY, US; 1 February 2022 (2022-02-01), LI M: "A**
**Review of Implant Intra-Body Communication",**
**XP002812915, Database accession no.**
**E20220911713305**
• **LI M: "A Review of Implant Intra-Body**
**Communication", JOURNAL OF BEIJING**
**INSTITUTE OF TECHNOLOGY (ENGLISH**
**EDITION) 20220201 BEIJING INSTITUTE OF**
**TECHNOLOGY CHN, vol. 31, no. 1, 1 February**
**2022 (2022-02-01), pages 1 - 29, DOI: 10.15918/**
**J.JBIT1004-0579.2021.076**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- **KAYHAN ATE ET AL: "Channel Characterization of Implantable Intrabody Communication through Experimental Measurements", ARXIV.ORG, CORNELL UNIVERSITY LIBRARY, 201 OLIN LIBRARY CORNELL UNIVERSITY ITHACA, NY 14853, 1 July 2024 (2024-07-01), XP091805685**
- **VIZZIELLO ANNA ET AL: "Experimental Channel Characterization of Human Body Communication Based on Measured Impulse Response", IEEE TRANSACTIONS ON COMMUNICATIONS, IEEE SERVICE CENTER, PISCATAWAY, NJ. USA, vol. 72, no. 7, 25 February 2024 (2024-02-25), pages 3970 - 3984, XP011976725, ISSN: 0090-6778, [retrieved on 20240226], DOI: 10.1109/TCOMM.2024.3370468**

**Description**

FIELD OF TECHNOLOGY

Embodiments of the present technology described herein generally relate to techniques to improve communication between implantable medical devices (IMDs). Embodiments of the present technology also relate to systems and devices that are configured to implement such techniques. Embodiments of the present technology described herein can also be used to improved conductive communication between an external device, which can also be referred to as an external medical device, and one or more IMDs.

BACKGROUND

Implant-to-Implant (i2i) communication is used by active implanted medical devices (AIMDs), such as leadless cardiac pacemakers (LCPs), to synchronize pacing and/or sensing events. An example of an existing solution for i2i communication is low frequency (kHz range) conductive telemetry. There are cases in which patients are implanted with both a dual chamber LCP system (which includes a pair of LCPs) and a non-vascular ICD (NV-ICD), such as, but not limited to, a subcutaneous implantable cardioverter defibrillator (S-ICD). In such cases, it may be useful for one or more of the LCPs to notify the NV-ICD about arrythmia events detected by the LCP(s) and for the NV-ICD to notify one or more of the LCPs to pause pacing during certain arrythmia events so that the NV-ICD can deliver shock therapy. Therefore, it would be useful to provide conductive telemetry communication between the LCP(s) and the NV-ICD that is compatible with existing conductive telemetry i2i communication between LCPs.

There are situations in which patients are exposed to noisy electromagnetic environments that may interfere with conductive telemetry. Such noise can be misinterpreted by one or more receiving devices as i2i communication signals and processed by device firmware, causing undefined behavior, such as change of programming parameters. Conductive telemetry communication signals transmitted from an LCP to an NV-ICD are especially subject to noise interference due to the generally weak signal reception at the NV-ICD.

US2022/370810 A1 describe methods, devices, and systems that provide improved communications between first and second IMDs remotely located relative to one another and capable of communicating using both conductive communication and RF communication. Such a method can include the first IMD using conductive communication to transmit message(s) intended for the second IMD, without using RF communication, during a first period of time that a first trigger event is not detected. The method can also include the first IMD detecting the first trigger event, and in response thereto, the first IMD using RF communication to transmit message(s) intended for the second IMD during a second period of time. Thereafter, in response to first IMD detecting a second trigger event, the first IMD uses conductive communication to transmit one or more messages intended for the second IMD, without using RF communication, during a third period of time.

SUMMARY

The present disclosure relates to a method for use by a first implantable medical device (IMD1) generating a first series of bits that are to be communicated from the IMD1 to a second implantable medical device (IMD2), and generating a second series of bits that are to be communicated from the IMD1 to a third implantable medical device (IMD3). The method also includes the IMD1 encoding the first bit and the second bit into a composite bit comprising a first time slot including the first bit and a second time slot including the second bit, wherein the IMD1 encoding the first bit and the second bit comprises the IMD1 encoding the second bit as either a first pseudo noise pulse sequence corresponding to a first bit value or a second pseudo noise pulse sequence corresponding to a second bit value, wherein the first pseudo noise pulse sequence and the second pseudo noise pulse sequence are orthogonal to one another. The method also includes the IMD1 transmitting the composite bit, e.g., using conductive communication.

The present disclosure relates to a method for use by a first implantable medical device (IMD1) generating a first series of bits that are to be communicated from the IMD1 to a second implantable medical device (IMD2), and generating a second series of bits that are to be communicated from the IMD1 to a third implantable medical device (IMD3). The method also includes the IMD1 encoding the first series of bits and the second series of bits into a series of composite bits, each composite bit of the series of composite bits comprising a first time slot and a second time slot, the first time slot of the composite bit including a respective bit of the first series of bits, and the second time slot of the composite bit including a respective bit of the second series of bits. In certain embodiments, encoding the second series of bits comprises encoding each bit of the second series of bits as either a first pseudo noise pulse sequence corresponding to a first bit value of the second series, or a second pseudo noise pulse sequence corresponding to a second bit value of the second series, wherein the first pseudo noise pulse sequence and the second pseudo noise pulse sequence are orthogonal to one another. The method also includes the IMD1 transmitting the series of composite bits, e.g., using conductive communication.

In accordance with certain examples, the method further comprises the IMD3: receiving the series of composite bits; and decoding the received series of composite bits to extract the second series of bits.

In accordance with certain examples, the IMD3 decoding the received series of composite bits to extract the second series of bits comprises the IMD3: multiplying each of the received composite bits by the first pseudo noise pulse sequence; integrating each of the received composite bits of the second time slot multiplied by the first pseudo noise pulse sequence over a duration; multiplying each of the received composite bits by the second pseudo noise pulse sequence; integrating each of the received composite bits multiplied by the second pseudo noise pulse sequence over the duration of the second time slot; mapping each of the integrated received composite bits multiplied by the first pseudo noise pulse sequence and each of the integrated received composite bits multiplied by the second pseudo noise pulse sequence to a respective in-phase channel value and a respective quadrature channel value; and determining, from a combination of the in-phase channel values and the quadrature channel values, the value of each of the bits of the second series of bits.

In accordance with certain examples, the IMD3 decoding the received series of composite bits to extract the second series of bits further comprises the IMD3: determining, from a combination of the in-phase channel values, the quadrature channel values, and the values of the bits of the second series of bits, that data content of the second series of bits as received and decoded is corrupted.

In accordance with certain examples, the IMD1 encoding the first series of bits comprises: encoding each bit of the first series of bits as either a flat waveform corresponding to a first bit value of the first series, or a series of pulses corresponding to a second bit value of the first series of bits.

In accordance with certain examples, the IMD1 encoding the first series of bits comprises: encoding each bit of the first series of bits as either a third pseudo noise pulse sequence corresponding to a first bit value of the first series, or a fourth pseudo noise pulse sequence corresponding to a second bit value of the first series, wherein the first pseudo noise pulse sequence, the second pseudo noise pulse sequence, the third pseudo noise pulse sequence, and the fourth pseudo noise pulse sequence are mutually orthogonal.

In accordance with certain examples, the method further comprises: the IMD1 maintaining a lookup table of a correspondence between bit values of the second series of bits and the first and second pseudo noise pulse sequences; and wherein the IMD1 encoding the first series of bits and the second series of bits comprises that IMD1 using the lookup table to translate each bit of second series to the corresponding pseudo noise pulse sequence.

In accordance with certain examples, the series of composite bits is a specified number of composite bits.

In accordance with certain examples, the method further comprises the IMD1 transmitting the series of composite bits using conductive communication, which comprises: transmitting a wake up pulse; and subsequent to transmitting the wake up pulse, transmitting the series of composite bits.

In accordance with certain examples, the IMD1 transmitting the series of composite bits using conductive communication further comprises: subsequent to transmitting the wake up pulse and prior to transmitting the series of composite bits, waiting a specified time period.

In accordance with certain examples, the series of composite bits comprises a plurality of frames, each of the frames comprising a plurality of the composite bits.

In accordance with certain examples, the method further comprises the IMD3 transmitting a signal intended for reception by the IMD1 using conductive communication, the signal comprising a further series of composite bits each comprising a first time slot including a waveform corresponding to data bit value and a second time slot not containing data.

In accordance with certain examples, the method further comprises the IMD1: receiving the signal intended for reception by the IMD1; and for each bit of the further series of composite bits, determining by the IMD1 the data value of the first time slot during the second time slot.

In accordance with certain examples, the IMD1 is a first leadless cardiac pacemaker (LCP1), the IMD2 is a second leadless cardiac pacemaker (LCP2), and the third device is a NV-ICD. In certain embodiments, the IMD2 is a type of NV-ICD also known as an S-ICD.

In accordance with certain examples, the first time slot precedes the second time slot in each of the composite bits of the series of composite bits.

Certain embodiments of the present technology are directed to an implantable medical device including conductive communication circuitry, that is configured to transmit a signal to a plurality of additional implantable medical devices, and a controller connected the conductive communication circuitry. The controller is configured to: generate a first series of bits that are to be communicated to a first of the additional implantable medical devices; generate a second series of bits that are to be communicated to a second of the additional implantable medical devices; encode the first series of bits and the second series of bits into a series of composite bits. Each composite bit of the series of composite bits comprising a first time slot and a second time slot, the first time slot of the composite bit including a respective bit of the first series of bits, and the second time slot of the composite bit including a respective bit of the second series of bits. To encode the first series of bits and the second series of bits, the controller is configured to encode each bit of the second series of bits as either a first pseudo noise pulse sequence corresponding to a first bit value of the second series, or a second pseudo noise pulse sequence corresponding to a second bit value of the second series, wherein the first pseudo noise pulse sequence and the second pseudo noise pulse sequence are orthogonal to one another. The controller is also configured to control the conductive communication circuitry to transmit the series of composite bits using conductive communication.

In accordance with certain embodiments of the present technology, to encode the first series of bits, the controller is further configured to: encode each bit of the first series of bits as either a flat waveform corresponding to a first bit value of the first series, or a series of pulses corresponding to a second bit value of the first series of bits.

In accordance with certain embodiments of the present technology, the controller is further configured to: maintain a lookup table of a correspondence between bit values of the second series of bits and the first and second pseudo noise pulse sequences; and wherein encoding the second series of bits comprises that IMD1 using the lookup table to translate each bit of second series to the corresponding pseudo noise pulse sequence.

In accordance with certain embodiments, the series of composite bits is a specified number of composite bits.

In accordance with certain embodiments, the controller is configured to control the conductive communication circuitry to transmit a wake up pulse, and subsequent to transmitting the wake up pulse, transmit the series of composite bits.

In accordance with certain embodiments, the controller is configured to control the conductive communication circuitry to wait a specified time period subsequent to transmitting the wake up pulse and prior to transmitting the series of composite bits.

In accordance with certain embodiments, the series of composite bits comprises a plurality of frames, each of the frames comprising a plurality of the composite bits.

In accordance with certain embodiments, the implantable medical device comprises a first leadless cardiac pacemaker (LCP1), the first of the plurality of additional implantable medical devices comprises a second leadless cardiac pacemaker (LCP2), and the second of the plurality of additional implantable medical devices.

In accordance with certain embodiments, the first time slot precedes the second time slot in each of the composite bits of the series of composite bits.

Certain embodiments of the present technology are directed to a system, comprising a plurality of implantable medical devices, including a first implantable medical device (IMD1), a second implantable medical device (IMD2), and a third implantable medical device (IMD3). The IMD1 configured to: generate a first series of bits that are to be communicated from the IMD1 to the IMD2; generate a second series of bits that are to be communicated from the IMD1 to the IMD3; encode the first series of bits and the second series of bits into a series of composite bits, each composite bit of the series of composite bits comprising a first time slot and a second time slot, the first time slot of the composite bit including a respective bit of the first series of bits, and the second time slot of the composite bit including a respective bit of the second series of bits. To encode the first series of bits and the second series of bits, the IMD 1 is configured to encode each bit of the second series of bits as either a first pseudo noise pulse sequence corresponding to a first bit value of the second series, or a second pseudo noise pulse sequence corresponding to a second bit value of the second series, wherein the first pseudo noise pulse sequence and the second pseudo noise pulse sequence are orthogonal to one another. The IMD1 is also configured to transmit the series of composite bits using conductive communication.

In accordance with certain embodiments of the present technology, the IMD3 is configured to: receive the series of composite bits; multiply each of the received composite bits by the first pseudo noise pulse sequence; integrate each of the received composite bits of the second time slot multiplied by the first pseudo noise pulse sequence over a duration; multiply each of the received composite bits by the second pseudo noise pulse sequence; integrate each of the received composite bits multiplied by the second pseudo noise pulse sequence over the duration of the second time slot; map each of the integrated received composite bits multiplied by the first pseudo noise pulse sequence and each of the integrated received composite bits multiplied by the second pseudo noise pulse sequence to a respective in-phase channel value and a respective quadrature channel value; and determine, from a combination of the in-phase channel values and the quadrature channel values, the value of each of the bits of the second series of bits.

In accordance with certain embodiments, in order to encode the first series of bits, the IMD1 is configured to encode each bit of the first series of bits as either a flat waveform corresponding to a first bit value of the first series, or a series of pulses corresponding to a second bit value of the first series of bits.

In accordance with certain embodiments, in order to encode the first series of bits, the IMD1 is configured to encode each bit of the first series of bits as either a third pseudo noise pulse sequence corresponding to a first bit value of the first series, or a fourth pseudo noise pulse sequence corresponding to a second bit value of the first series, wherein the first pseudo noise pulse sequence, the second pseudo noise pulse sequence, the third pseudo noise pulse sequence, and the fourth pseudo noise pulse sequence are mutually orthogonal.

In accordance with certain embodiments, in order to encode the first series of bits, the IMD1 is configured to maintain a lookup table of a correspondence between bit values of the second series of bits and the first and second pseudo noise pulse sequences, and encode the first series of bits and the second series of bits using the lookup table to translate each bit of second series to the corresponding pseudo noise pulse sequence.

In accordance with certain embodiments, the series of composite bits is a specified number of composite bits.

In accordance with certain embodiments, in order to transmit the series of composite bits using conductive communication the IMD1 is configured to transmit a wake up pulse, and subsequent to transmitting the wake up pulse, transmit the series of composite bits.

In accordance with certain embodiments, in order to transmit the series of composite bits using conductive communication the IMD1 is configured to wait a specified period of tie subsequent to transmitting the wake up pulse and prior to transmitting the series of composite bits.

In accordance with certain embodiments, the series of composite bits comprises a plurality of frames, each of the frames comprising a plurality of the composite bits.

In accordance with certain embodiments, the IMD3 is configured to transmit a signal intended for reception by the IMD1 using conductive communication, the signal comprising a further series of composite bits each comprising a first time slot including waveform corresponding to a data bit value and a second time slot not containing data.

In accordance with certain embodiments, the IMD1 is configured to receive the signal intended for reception by the IMD1, and for each bit of the further series of composite bits, determine the data value of the first time slot during the second time slot.

In accordance with certain embodiments, the IMD1 comprises a first leadless cardiac pacemaker (LCP1), the IMD2 comprises a second leadless cardiac pacemaker (LCP2), and the third device comprises a non-vascular implantable cardioverter defibrillator (NV-ICD).

In accordance with certain embodiments, the first time slot precedes the second time slot in each of the composite bits of the series of composite bits.

The present disclosure relates to methods for use by a first implantable medical device (IMD1) generating a first series of bits that are to be communicated from the IMD1 to a second implantable medical device (IMD2). The method also includes the IMD1 encoding each bit of the first series of bits as either a first pseudo noise pulse sequence corresponding to a first bit value of the first series, or a second pseudo noise pulse sequence corresponding to a second bit value of the first series, to thereby produce an encoded series of bits, wherein the first pseudo noise pulse sequence and the second pseudo noise pulse sequence are orthogonal to one another. The method additionally includes the IMD1 transmitting the encoded series of bits using conductive communication.

In accordance with certain examples, the method further comprises the IMD2: receiving the encoded series of bits; and decoding the received series of encoded bits to extract the first series of bits.

In accordance with certain examples, the IMD2 decoding the received series of encoded bits to extract the first series of bits comprises the IMD2: multiplying each received bit of the received series of encoded bits by the first pseudo noise pulse sequence; integrating each of the received series of encoded bits multiplied by the first pseudo noise pulse sequence over a duration of the encoded bit; multiplying each received bit of the received series of encoded bits by the second pseudo noise pulse sequence; integrating each of the received series of encoded bits multiplied by the second pseudo noise pulse sequence over the duration of the encoded bit; mapping each of the integrated received series of encoded bits multiplied by the first pseudo noise pulse sequence and each of the integrated received series of encoded bits multiplied by the second pseudo noise pulse sequence to a respective in-phase channel value and a respective quadrature channel value; and determining, from a combination of the in-phase channel values and the quadrature channel values, the value of each of the bits of the first series of bits.

In accordance with certain examples, the IMD2 decoding the received series of encoded bits to extract the first series of bits further comprises the IMD2: determining, from a combination of the in-phase channel values, the quadrature channel values, and the determined values of the bits of the first series of bits, that data content of the first series of bits as received and decoded is corrupted.

In accordance with certain examples, the method further comprises: prior to the IMD1 encoding each bit of the first series of bits as either a first pseudo noise pulse sequence or a second pseudo noise pulse sequence, selecting the IMD2 from a plurality of implantable medical devices, each of the plurality of implantable medical devices having a corresponding one of a plurality of mutually orthogonal first and second pseudo noise pulse sequences; and wherein the IMD1 encoding each bit of the first series of bits as either the first pseudo noise pulse sequence or the second pseudo noise pulse sequence comprises encoding each bit of the first series of bits as either the first pseudo noise pulse sequence or the second pseudo noise pulse sequence corresponding to the selected IMD2.

In accordance with certain examples, the method further comprises: the IMD1 maintaining a look up table of the corresponding mutually orthogonal first and second pseudo noise pulse sequences for each of the plurality of implantable medical devices; and the IMD1 selecting the corresponding one of a plurality of mutually orthogonal first and second pseudo noise pulse sequences from the look up table.

In accordance with certain examples, the method further comprises prior to the IMD1 transmitting the encoded series of bits using conductive communication, the IMD1 transmitting an initial series of bits encoded as either a third pseudo noise pulse sequence or a fourth pseudo noise pulse sequence, the first, second, third, and fourth pseudo noise pulse sequences being mutually orthogonal, the initial series of bits specifying: the IMD2 among a plurality of implantable medical devices; and the first and second pseudo noise pulses.

In accordance with certain examples, the method further comprises the IMD2: receiving the initial series of bits; and determining whether the initial series of bits specifies the IMD2.

In accordance with certain examples, the method further comprises the IMD2: in response to the initial series of bits specifying the IMD2, decoding the first bit values of the first series.

In accordance with certain examples, the method further comprises the IMD2: subsequent to decoding the first bit values of the first series, replying to the IMD1 using a signal encoded with the first and second pseudo noise pulses.

In accordance with certain examples, the plurality of implantable medical devices including a third implantable medical device (IMD3), the method further comprising the IMD3: receiving the initial series of bits; determining whether the initial series of bits specifies the IMD3; and in response to the initial series of bits not specifying the IMD3, not decoding the first bit values of the first series.

In accordance with certain examples, the first series of bits is a specified number of bits.

In accordance with certain examples, the IMD1 transmitting the first series of bits using conductive communication comprises: transmitting a wake up pulse; and subsequent to transmitting the wake up pulse, transmitting the first series of bits.

In accordance with certain examples, the IMD1 transmitting the first series of bits using conductive communication further comprises: subsequent to transmitting the wake up pulse and prior to transmitting the first series of bits, waiting a specified time period.

In accordance with certain embodiments of the present technology, the IMD1 is a first leadless cardiac pacemaker (LCP1) and the IMD2 is a NV-ICD. In certain embodiments, the IMD2 is a type of NV-ICD also known as an S-ICD.

In accordance with certain embodiments of the present technology, the IMD1 is a first leadless cardiac pacemaker (LCP1) and the IMD2 is a second leadless cardiac pacemaker (LCP2).

Certain embodiments of the present technology are directed to a system comprising a plurality of implantable medical devices, including a first implantable medical device (IMD1) and a second implantable medical device (IMD2). The IMD1 is configured to: generate a first series of bits that are to be communicated from the IMD1 to the IMD; encode each bit of the first series of bits as either a first pseudo noise pulse sequence corresponding to a first bit value of the first series, or a second pseudo noise pulse sequence corresponding to a second bit value of the first series, to thereby produce an encoded series of bits, wherein the first pseudo noise pulse sequence and the second pseudo noise pulse sequence are orthogonal to one another; and to transmit the encoded series of bits using conductive communication.

In accordance with certain embodiments of the present technology, the IMD1 is further configured to: prior to encoding each bit of the first series of bits as either a first pseudo noise pulse sequence or a second pseudo noise pulse sequence, select the IMD2 from a plurality of implantable medical devices, each of the plurality of implantable medical devices having a corresponding one of a plurality of mutually orthogonal first and second pseudo noise pulse sequences; and wherein to encode each bit of the first series of bits as either the first pseudo noise pulse sequence or the second pseudo noise pulse sequence, the IMD1 is further configured to encode each bit of the first series of bits as either the first pseudo noise pulse sequence or the second pseudo noise pulse sequence corresponding to the selected IMD2.

In accordance with certain embodiments of the present technology, the IMD1 is further configured to: prior to transmitting the encoded series of bits using conductive communication, transmit an initial series of bits encoded as either a third pseudo noise pulse sequence or a fourth pseudo noise pulse sequence, the first, second, third, and fourth pseudo noise pulse sequences being mutually orthogonal, the initial series of bits specifying: the IMD2 among a plurality of implantable medical devices; and the first and second pseudo noise pulse sequences.

In accordance with certain embodiments, in order to encode the first series of bits, the IMD1 is configured to encode each bit of the first series of bits as either a flat waveform corresponding to a first bit value of the first series, or a series of pulses corresponding to a second bit value of the first series of bits.

In accordance with certain embodiments, in order to encode the first series of bits, the IMD1 is configured to encode each bit of the first series of bits as either a third pseudo noise pulse sequence corresponding to a first bit value of the first series, or a fourth pseudo noise pulse sequence corresponding to a second bit value of the first series, wherein the first pseudo noise pulse sequence, the second pseudo noise pulse sequence, the third pseudo noise pulse sequence, and the fourth pseudo noise pulse sequence are mutually orthogonal.

In accordance with certain embodiments, in order to encode the first series of bits, the IMD1 is configured to maintain a lookup table of a correspondence between bit values of the second series of bits and the first and second pseudo noise pulse sequences, and encode the first series of bits and the second series of bits using the lookup table to translate each bit of second series to the corresponding pseudo noise pulse sequence.

In accordance with certain embodiments, the series of composite bits is a specified number of composite bits.

In accordance with certain embodiments, in order to transmit the series of composite bits using conductive communication the IMD1 is configured to transmit a wake up pulse, and subsequent to transmitting the wake up pulse, transmit the series of composite bits.

In accordance with certain embodiments, in order to transmit the series of composite bits using conductive communication the IMD1 is configured to wait a specified period of tie subsequent to transmitting the wake up pulse and prior to transmitting the series of composite bits.

In accordance with certain embodiments, the series of composite bits comprises a plurality of frames, each of the frames comprising a plurality of the composite bits.

In accordance with certain embodiments, the first time slot precedes the second time slot in each of the composite bits of the series of composite bits.

Certain embodiments of the present technology are directed to an implantable medical device including conductive communication circuitry configured to transmit a signal from a first implantable medical device (IMD1) to a second implantable medical device (IMD2); and a controller connected the conductive communication circuitry. The controller is configured to: generate a first series of bits that are to be communicated from the IMD1 to the IMD2; encode each bit of the first series of bits as either a first pseudo noise pulse sequence corresponding to a first bit value of the first series, or a second pseudo noise pulse sequence corresponding to a second bit value of the first series, wherein the first pseudo noise pulse sequence and the second pseudo noise pulse sequence are orthogonal to one another; and control the conductive communication circuitry to transmit the series of composite bits using conductive communication.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

BRIEF DESCRIPTION OF THE DRAWINGS

Embodiments of the present technology relating to both structure and method of operation may best be understood by referring to the following description and accompanying drawings, in which similar reference characters denote similar elements throughout the several views:

FIG. 1 illustrates a system that includes a plurality of implantable medical devices (IMDs) that are implanted in a patent.
FIG. 2 is a high level block diagram of an example LCP.
FIG. 3 illustrates an example form factor for an LCP.
FIG. 4 is a high level block diagram illustrating example details of an implantable cardioverter defibrillator (ICD) that is configured to communicate with one or more other IMDs implanted within a patient using conductive communication.
FIG. 5 illustrates an example of a transmitting signal waveform of one bit from one LCP to another LCP (in a time slot 1) and also to an NV-ICD (in a time slot 2).
FIG. 6 illustrates an example of a transmitting signal waveform of one bit from an NV-ICD to an LCP.
FIG. 7 illustrates an embodiment for a pseudo noise pulse encoded data frame format layout.
FIG. 8 illustrates an example of waveforms for an orthogonal pair of pseudo noise pulse sequences $\{PN_0, PN_1\}$ that meet the pseudo noise conditions.
FIG. 9 is a block diagram of an embodiment for encoding of the two series of bits to be transmitted into composite bits as illustrated FIG. 5.
FIG. 10 is a flowchart of an embodiment for the encoding of signals for implant-to-implant communication from a first device to a pair of other devices using the format of FIGs. 5-9.
FIG. 11 is a diagram of an embodiment for a pseudo noise pulse demodulation system.
FIG. 12 illustrates a decoding constellation diagram for the I-Q channels in FIG. 11 at the receiving IMD, e.g., an NV-ICD.
FIG. 13 illustrates an embodiment for a data frame format for a public-private $\{PN_0, PN_1\}$ pair method.
FIG. 14 is a flowchart of an embodiment of the public-private $\{PN_0, PN_1\}$ pair method.
FIG. 15 illustrates an embodiment for a decentralized implant-to-implant communication network using a public-private $\{PN_0, PN_1\}$ pair method.
FIG. 16 illustrates an embodiment for a master/slave implant-to-implant communication network with a public-private $\{PN_0, PN_1\}$ pair arrangement.
FIG. 17 illustrates an embodiment for a data frame format for a private $\{PN_0, PN_1\}$ pair method.
FIG. 18 is a flowchart of an embodiment of the private $\{PN_0, PN_1\}$ pair method.
FIG. 19 illustrates an embodiment for a decentralized implant-to-implant communication network using a private $\{PN_0, PN_1\}$ pair method for a three device embodiment.
FIG. 20 illustrates an embodiment for a master/slave implant-to-implant communication network with a public-private $\{PN_0, PN_1\}$ pair arrangement.
FIGs. 21 and 22 respectively illustrate pseudo noise encoding/decoding without noise and with additive Gaussian noise.

DETAILED DESCRIPTION

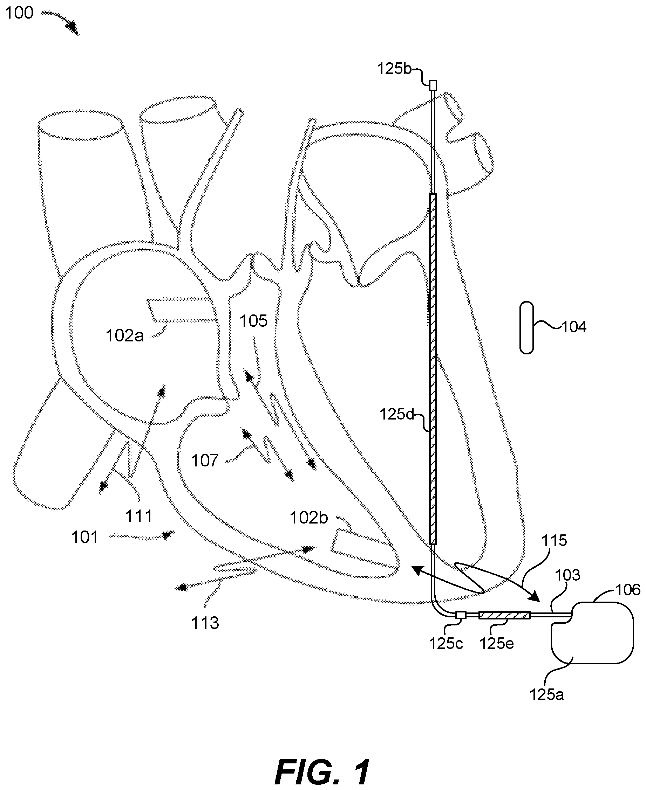

Embodiments of the present technology can be used to improve conductive communication between IMDs, e.g., between multiple LCPs and/or between an implantable cardioverter defibrillator and one or more LCPs, but is not limited thereto. Certain embodiments provide conductive telemetry communication between LCPs and a NV-ICD that is compatible with certain LCP to LCP communication systems. The communication link from an NV-ICD to the LCPs can use the same information encoding/decoding techniques as certain existing LCP implant-to-implant communication systems, while the communication link from an LCP to an NV-ICD uses a pseudo random noise information encoding/decoding technique that has enhanced performance for noise resistance. FIGS. 1-4 show an example cardiac pacing system with which embodiments of the present technology can be used, wherein pacing and sensing operations can be performed by multiple IMDs. Such IMDs may include, for example, one or more LCPs, an ICD, such as a NV-ICD, and/or other implantable devices such as an insertable cardiac monitor (ICM) or a pulmonary artery pressure monitoring device. Where the system includes an ICD, the system is also capable of performing defibrillation.

A conductive communication pulse, as the term is used herein, refers to a voltage pulse that is generated by a device (e.g., by a pulse generator thereof) and emitted between at least a pair of electrodes that corresponds to a conductive communication vector, such that the conductive communication pulse is conducted through patient tissue and may be sensed by at least another pair of electrodes of or electrically coupled to a second device. A conductive communication message, as the term is used herein, can include a sequence of such conductive communication pulses that collectively impart information, e.g., after being decoded and/or demodulated. A conductive communication signal, as the term is used herein, can include one or more conductive communication pulses that collectively make up one or more conductive communication messages, or collectively make up at least a portion of at least one conductive communication message, i.e., a conductive communication signal includes one or more conductive communication pulses. Conductive communication can also be referred to interchangeably as conducted communication. Similarly, conductive communication pulses can also be referred to interchangeably as conducted communication pulses.

FIG. 1 illustrates a system 100 that is configured to be at least partially implanted in a heart 101. The system 100 includes LCPs 102a and 102b located in different chambers of the heart 101. The LCP 102a is located in a right atrium, while LCP 102b is located in a right ventricle. The LCPs 102a and 102b can communicate with one another to inform one another of various local physiologic activities, such as local intrinsic events, local paced events, and/or the like. The LCPs 102a and 102b may be constructed in a similar manner, but operate differently based upon which chamber LCP 102a or 102b is located. The LCPs 102a and 102b may sometimes be referred to collectively herein as the LCPs 102, or individually as an LCP 102.

In certain embodiments, the LCPs 102a and 102b communicate with one another, and/or with an ICD 106, and/or with an additional device such as an insertable cardiac monitor (ICM) or external device, by conductive communication through the same electrodes that are used for sensing and/or delivery of pacing therapy. While not shown (and not preferred, since it would increase the size and power consumption of the LCPs 102a and 102b), the LCPs 102a and 102b can potentially include an antenna and/or telemetry coil that would enable them to communicate with one another, the ICD 106 and/or a non-implanted device using RF and/or inductive communication. While only two LCPs 102 are shown in FIG. 1, it is possible that more than two LCPs can be implanted in a patient. For example, to provide for bi-ventricular pacing and/or cardiac resynchronization therapy (CRT), in addition to having LCPs implanted in or on the right atrial (RA) chamber and the right ventricular (RV) chamber, a further LCP can be implanted in or on the left ventricular (LV) chamber. It is also possible that a single LCP be implanted within a patient, e.g., in or on the RV chamber, the RA chamber, or the LV chamber, but not limited thereto. It would also be possible for more than one LCP to be implanted in or on a same cardiac chamber. The ICD 106 is shown as being connected to a lead 103, on which is located multiple electrodes. In FIG. 1, the electrically conductive canister (aka housing or case) of the ICD (or a portion thereof) provides a can or case electrode 125a. The lead 103 is shown as including a distal ring (or tip) electrode 125b, a proximal ring electrode 125c, a distal coil electrode 125d (also known as a parasternal coil electrode 125d) and a proximal coil electrode 125e (also known as a traverse coil electrode 125e). The lead 103 can include more or less electrodes than shown. It is also possible that the ICD 106 be connected to more than one lead and/or to a patch electrode.

Each LCP 102a, 102b uses two or more electrodes located within, on, or within a few centimeters of the housing of the pacemaker, for pacing and sensing at the cardiac chamber, and additionally for bidirectional conductive communication with one another and/or with the ICD 106.

Each LCP 102a, 102b and/or other type of IMD can transmit an advertisement sequence of pulses using at least two electrodes of the IMD (e.g., LCP) from time to time so that an external device (e.g., an external programmer, or a remote monitor, not shown in FIG. 1) that has or is communicatively coupled to external electrodes that are in contact with the patient (within which the LCP(s) and/or other IMD(s) is/are implanted) can detect the presence of the IMD(s) and optionally establish a communication session with one or more IMD(s). For a more specific example, an LCP (or other type of IMD) can transmit an advertisement sequence of pulses every specified number of cardiac cycles (e.g., every eight cardiac cycles), or every specified period of time (e.g., every 5 seconds), but not limited thereto. In accordance with certain embodiments, the advertisement sequence of pulses is a predetermined sequence of pulses that indicates to an external device (e.g., an external programmer, or a remote monitor) that an LCP (or other type of IMD) is implanted within a patient. The advertisement sequence of pulses can also be referred to as a sniff sequence of pulses, or more succinctly as a sniff.

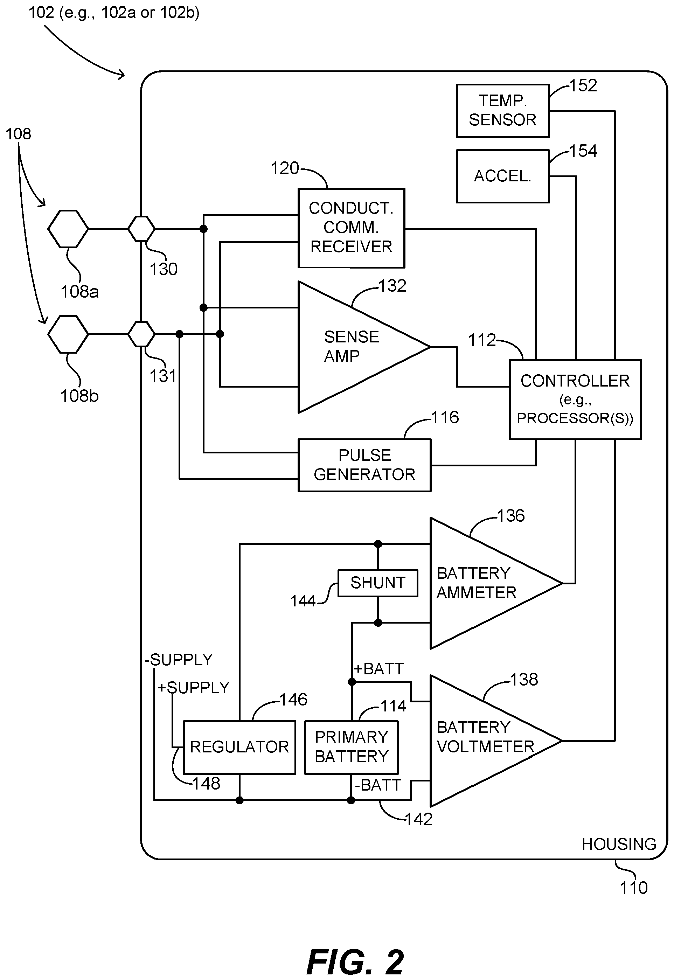

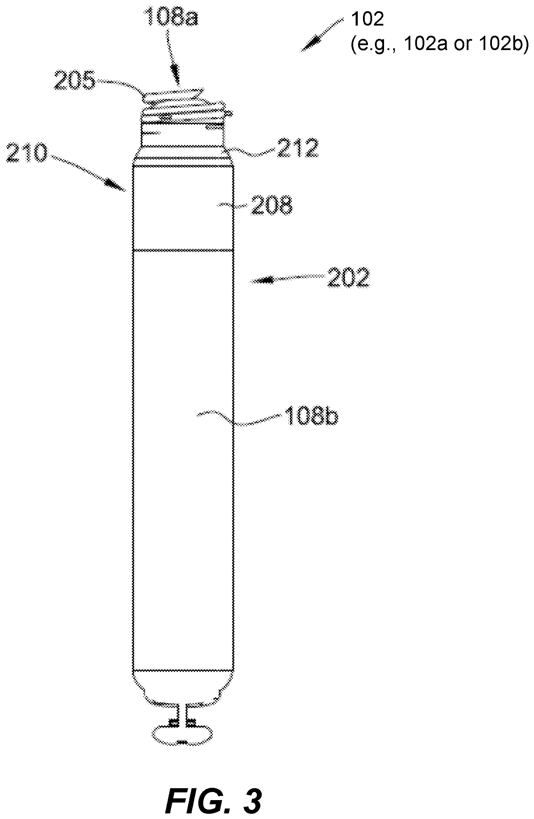

Referring to FIG. 2, a block diagram shows an example embodiment for portions of the electronics within the LCPs 102a, 102b configured to provide conductive communication through the same electrodes that are used for cardiac pacing and/or sensing. Each of the LCPs 102a, 102b includes two leadless electrodes configured for delivering cardiac pacing pulses, sensing evoked and/or natural cardiac electrical signals, and unidirectional and/or bidirectional conductive communication. In FIG. 2 (and FIG. 3) the two electrodes shown therein are labeled 108a and 108b. Such electrodes can be referred to collectively as the electrodes 108, or individually as an electrode 108. An LCP 102, or other type of IMD, can include more than two electrodes, depending upon implementation. However, unless stated otherwise, for much of the following description it is assumed that each LCP 102 includes only two electrodes, aka, a pair of electrodes.

In FIG. 2, each of the LCPs 102a, 102b is shown as including a conductive communication receiver 120 that is coupled to the electrodes 108a and 108b and configured to receive conductive communication signals from the other LCP 102 and/or the ICD 106, but not limited thereto. The electrode 108a and 108b can be referred to collectively as the electrodes 108, or individually as an electrode 108. The conductive communication receiver 120 and the electrodes 108 can also be used to receive conductive communication signals from external devices (not shown). Although one receiver 120 is depicted in FIG. 2, in other embodiments, each LCP 102a, 102b may also include one or more additional receivers. For example, each LCP 102 can include a low frequency (LF) receiver and a high frequency (HF) receiver, e.g., as shown in and described with reference to FIG. 2 of US2022/0370810, titled "Implant to Implant Communication for use with Implantable Medical Devices". For example, in US2022/0370810, the receivers 120 and 122 in FIG. 1 can be referred to, respectively, as a low frequency (LF) receiver 120 and a high frequency (HF) receiver 122, because the receiver 120 is configured to monitor for one or more signals within a relatively low frequency range (e.g., below 100 KHz) and the receiver 122 is configured to monitor for one or more signals within a relatively high frequency range (e.g., above 100 KHz). In certain embodiments, the receiver 120 (and more specifically, at least a portion thereof) is always enabled and monitoring for a wakeup notice, which can simply be a wakeup pulse, within a specific low frequency range (e.g., between 1 KHz and 100 KHz); and the receiver 122 is selectively enabled by the receiver 120. The receiver 120 is configured to consume less power than the receiver 122 when both the first and second receivers are enabled. Accordingly, the receiver 120 can also be referred to as a low power receiver 120, and the receiver 122 can also be referred to as a high power receiver 122. The low power receiver 120 is incapable of receiving signals within the relatively high frequency range (e.g., above 100 KHz), but consumes significantly less power than the high power receiver 122. This way the low power receiver 120 is capable of always monitoring for a wakeup notice without significantly depleting the battery (e.g., 114) of the LP. In accordance with certain embodiments, the high power receiver 122 is selectively enabled by the low power receiver 120, in response to the low power receiver 120 receiving a wakeup notice, so that the high power receiver 122 can receive the higher frequency signals, and thereby handle higher data throughput needed for effective i2i communication without unnecessarily and rapidly depleting the battery of the LP (which the high power receiver 122 may do if it were always enabled). As will be described in additional detail below, the pulse generator 116 can function as a transmitter that transmits conductive communication signals using the electrodes 108, under the control of the controller 112. The controller 112 can modulate and/or encode such pulses, or modulation and/or encoding of pulses can be performed external to the controller 112, yet at least in part under the control of the controller 112, depending on the specific implementation.

In certain embodiments, the LCPs 102a, 102b may communicate over multiple communication channels as indicated in FIG. 1. For example, in some embodiments, the LCPs 102a, 102b may communicate over one common communication channel 107, respectively communicate with the ICD 106 over channels 105 and 115, and respectively communicate with other devices (e.g., an ICM or an external device) over channels 111 and 113. More specifically, the LCPs 102a and 102b can communicate conductively over a common physical channel via the same electrodes 108 that are also used to deliver pacing pulses. Usage of the electrodes 108 for conductive communication enables the one or more LCPs 102a, 102b to perform antenna-less and inductive coil-less communication. Where multiple implantable devices (such as the LCPs 102a and 102b) communicate with one another using conductive communication, such conductive communication can be referred to as implant-to-implant (i2i) conductive communication, or more succinctly, as i2i conductive communication.

Optionally, an LCP (or other IMD) that receives any conductive communication signal from another LCP (or other IMD) or from a non-implanted device (e.g., a programmer or other external device) may transmit a receive acknowledgement indicating that the receiving LCP (or other IMD, or external device) received the conductive communication signal. In certain embodiments, where an IMD expects to receive a conductive communication signal within a window, and fails to receive the conductive communication signal within the window, the IMD may transmit a failure-to-receive acknowledgement indicating that the receiving IMD failed to receive the conductive communication signal. Other variations are also possible and within the scope of the embodiments described herein. Each conductive communication signal can include one or more sequences of conductive communication pulses. In accordance with certain embodiments, conductive communication pulses are preferably transmitted during cardiac refractory periods that are identified or detected by the LCP(s) and/or other IMD(s). In accordance with certain embodiments, conductive communication pulses are preferably subthreshold, i.e., they are below the cardiac capture threshold for the patient.

The LCPs 102a, 102b can exchange event messages within i2i conductive communication signals to enable synchronized therapy and additional supportive features (e.g., measurements, etc.). To maintain synchronous therapy, each of the LCPs 102a, 102b is made aware (through the event messages) when an event occurs in the chamber containing the other LCP 102a, 102b. For example, event messages can be used by one LCP to inform the other LCP (and/or an NV-ICD) of intrinsic events sensed by the LP, paced events caused by the LP, and/or the like. Example additional details of i2i event messages that are sent between LCPs 102 are provided in US 2022/0370810.

For synchronous event signaling, LCPs 102a and 102b may maintain synchronization and regularly communicate at a specific interval. Synchronous event signaling allows the transmitter (e.g., pulse generator 116) and receiver in each LCP 102a,102b to use limited (or minimal) power as each LCP 102a, 102b is only powered for a small fraction of the time in connection with transmission and reception. For example, LCP 102a, 102b may transmit/receive (Tx/Rx) communication messages in time slots having duration of 10-20 μs, where the Tx/Rx time slots occur periodically (e.g., every 10-20 ms). Such time slots can also be referred to as windows.

Still referring to FIG. 2, each LCP 102a, 102b is shown as including a controller 112 and a pulse generator 116. The controller 112 can include, e.g., a microprocessor (or equivalent control circuitry), RAM and/or ROM memory, logic and timing circuitry, state machine circuitry, and I/O circuitry, but is not limited thereto. The controller 112 can further include, e.g., timing control circuitry to control the timing of the stimulation pulses (e.g., pacing rate, atrio-ventricular (AV) delay, atrial interconduction (A-A) delay, or ventricular interconduction (V-V) delay, etc.). Such timing control circuitry may also be used for the timing of refractory periods, blanking intervals, noise detection windows, evoked response windows, alert intervals, marker channel timing, and so on. The controller 112 can further include other dedicated circuitry and/or firmware/software components that assist in monitoring various conditions of the patient's heart and managing pacing therapies. The controller 112 and the pulse generator 116 may be configured to transmit event messages, via the electrodes 108, in a manner that does not inadvertently capture the heart in the chamber where LCP 102a, 102b is located, such as when the associated chamber is not in a refractory state. In addition, a LCP 102a, 102b that receives an event message may enter an "event refractory" state (or event blanking state) following receipt of the event message. The event refractory/blanking state may be set to extend for a determined period of time after receipt of an event message in order to avoid the receiving LCP 102a, 102b from inadvertently sensing another signal as an event message that might otherwise cause retriggering. For example, the receiving LCP 102a, 102b may detect a measurement pulse from another LCP 102a, 102b or an external device.

In some embodiments, an individual LCP 102 can comprise a hermetic housing 110 configured for placement on or attachment to the inside or outside of a cardiac chamber and at least two leadless electrodes 108 proximal to the housing 110 and configured for conductive communication with at least one other device within or outside the body. Depending upon the specific implementation, and/or the other device with which an LCP is communicating, the conductive communication may be unidirectional or bidirectional.

FIG. 2 depicts an example LCP 102a (or 102b) and shows the LCP's functional elements substantially enclosed in a hermetic housing 110. The LCP 102a (or 102b) has at least two electrodes 108 located within, on, or near the housing 110, for delivering pacing pulses to and sensing electrical activity from the muscle of the cardiac chamber, and for conductive communication with at least one other device within or outside the body. Hermetic feedthroughs 130, 131 conduct electrode signals through the housing 110. The housing 110 contains a primary battery 114 to supply power for pacing, sensing, and communication. The housing 110 also contains a sense amplifier 132 for sensing cardiac activity from the electrodes 108, a receiver 120 (as noted above) for receiving information from at least one other device via the electrodes 108, and the pulse generator 116 for generating pacing pulses for delivery via the electrodes 108 and also for transmitting information to at least one other device via the electrodes 108. The housing 110 can further contain circuits for monitoring device health, for example an optional battery current monitor 136 and an optional battery voltage monitor 138, and can contain circuits for controlling operations in a predetermined manner.

The electrodes 108 can be configured to communicate bidirectionally among the multiple leadless cardiac pacemakers and/or the implanted ICD 106 to coordinate pacing pulse delivery and optionally other therapeutic or diagnostic features using messages that identify an event at an individual pacemaker originating the message and a pacemaker receiving the message can react as directed by the message depending on the origin of the message. An LCP 102a, 102b that receives the event message reacts as directed by the event message depending on the message origin or location. In some embodiments or conditions, the two or more leadless electrodes 108 can be configured to communicate bidirectionally among the one or more LCPs and/or the ICD 106 and transmit data including designated codes for events detected or created by an individual pacemaker. Individual pacemakers can be configured to issue a unique code corresponding to an event type and a location of the sending pacemaker. The electrodes can also be used to transmit and/or receive conductive communication signals from an external device.

As shown in FIG. 2, each LCP 102a, 102b can comprise two (or more) leadless electrodes 108 configured for delivering cardiac pacing pulses, sensing evoked and/or natural cardiac electrical signals, and bidirectionally communicating with one another and/or the co-implanted ICD 106. As shown in FIG. 2, the primary battery 114 has positive terminal 140 and negative terminal 142. Current from the positive terminal 140 of primary battery 114 flows through an optional shunt 144 to an optional regulator circuit 146 to create a positive voltage supply 148 suitable for powering the remaining circuitry of the pacemaker 102. The optional shunt 144 enables the optional battery current monitor 136 to provide the controller 112 with an indication of battery current drain and indirectly of device health. The illustrative power supply can be a primary battery 114. The LCP is also shown as including an optional temperature sensor 152 and an optional accelerometer 154.

In various embodiments, each LCP 102a, 102b can manage power consumption to draw limited power from the battery, thereby reducing device volume. Each circuit in the LCP 102 can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit is throttled to recharge the tank capacitor at constant power from the battery.

In some embodiments, the controller 112 in one LCP 102 can access signals on the electrodes 108 and can examine output pulse duration from another pacemaker for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller 112 can examine output pulse waveform from another leadless cardiac pacemaker for usage as a signature for determining triggering information validity and, for a signature arriving within predetermined limits, activating delivery of a pacing pulse following a predetermined delay of zero or more milliseconds.

FIG. 3 shows an example form factor of an LCP 102a, 102b. The LCP can include a hermetic housing 202 (110) with electrodes 108a and108b disposed thereon. As shown, electrode 108a can be separated from but surrounded partially by a fixation mechanism 205, and the electrode 108b can be disposed on the housing 202. The fixation mechanism 205 can be a fixation helix, a plurality of hooks, barbs, or other attaching features configured to attach the pacemaker to tissue, such as heart tissue. The electrodes 108a and 108b are examples of the electrodes 108 shown in and discussed above with reference to FIG. 2. The housing can also include an electronics compartment 210 within the housing that contains the electronic components necessary for operation of the pacemaker, including, e.g., a pulse generator, receiver, a battery, and a processor for operation. The hermetic housing 202 can be adapted to be implanted on or in a human heart, and can be cylindrically shaped, rectangular, spherical, or any other appropriate shapes, for example. The housing can comprise a conductive, biocompatible, inert, and anodically safe material such as titanium, 316L stainless steel, or other similar materials. The housing 202 can further comprise an insulator disposed on the conductive material to separate electrodes 108a and 108b. The insulator can be an insulative coating on a portion of the housing between the electrodes, and can comprise materials such as silicone, polyurethane, parylene, or another biocompatible electrical insulator commonly used for implantable medical devices. In the embodiment of FIG. 3, a single insulator 208 is disposed along the portion of the housing between electrodes 108a and 108b. In some embodiments, the housing itself can comprise an insulator instead of a conductor, such as an alumina ceramic or other similar materials, and the electrodes can be disposed upon the housing.

As shown in FIG. 3, the pacemaker can further include a header assembly 212 to isolate electrodes 108a and 108b. The header assembly 212 can be made from PEEK, tecothane or another biocompatible plastic, and can contain a ceramic to metal feedthrough, a glass to metal feedthrough, or other appropriate feedthrough insulator as known in the art. The term metal, as used herein, also encompasses alloys that are electrically conductive. The electrodes 108a and 108b can comprise pace/sense electrodes, or return electrodes. A low-polarization coating can be applied to the electrodes, such as sintered platinum, platinum-iridium, iridium, iridium-oxide, titanium-nitride, carbon, or other materials commonly used to reduce polarization effects, for example. In FIG. 3, the electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A helical fixation mechanism 205, can enable insertion of the device endocardially or epicardially through a guiding catheter. A torqueable catheter can be used to rotate the housing and force the fixation device into heart tissue, thus affixing the fixation device (and also the electrode 108a in FIG. 2) into contact with stimulable tissue. Electrode 108b can serve as an indifferent electrode for sensing and pacing. The fixation mechanism may be coated partially or in full for electrical insulation, and a steroid-eluting matrix may be included on or near the device to minimize fibrotic reaction, as is known in conventional pacing electrodeleads.

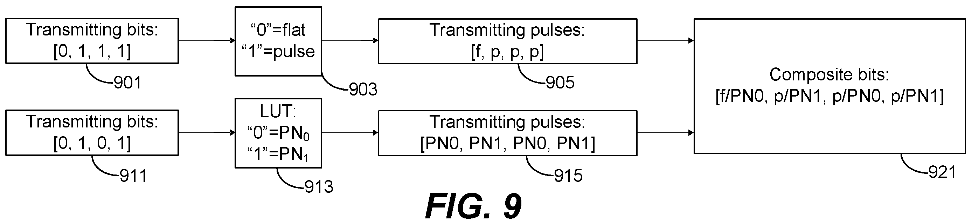

In FIGS. 1-3, each of the LCPs 102 was shown and described as including two electrodes 108. In alternative embodiments, one or more of the LCPs 102 can include more than two electrodes, e.g., three electrodes 108. Where an LCP 102 includes at least three electrodes, the LCP 102 can perform conductive communication using a plurality of different conductive communication vectors, wherein each of the plurality of different conductive communication vectors comprises a different combination of the at least three electrodes. Examples of an LCP that includes at least three electrodes are described with reference to FIGS. 9A and 9B of commonly assigned U.S. Patent Application No. 16/241,389, titled "Leadless Pacemakers and Methods for Use Therewith," filed January 7, 2019, and published as US 2020/0215536 A1.

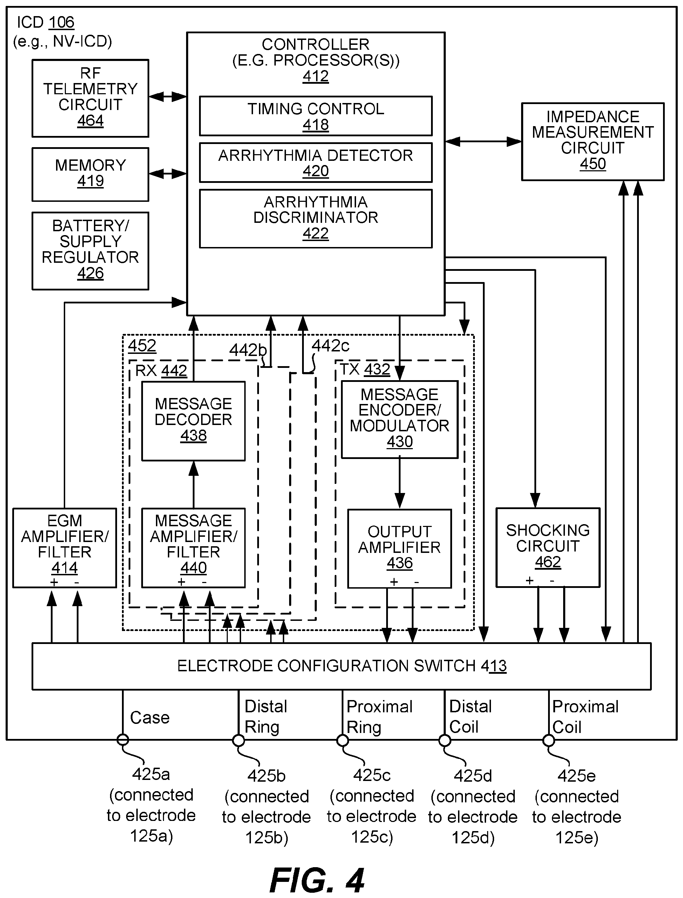

Referring to FIG. 4, shown therein is an example block diagram of the ICD 106 that can communicate with one or more other IMDs (e.g., LCPs) within a patient using conductive communication, and/or to communicate with an external device using conductive communication and/or RF communication. For the purpose of this discussion, it is presumed that the ICD 106 is a NV-ICD, such as, but not limited to, a subcutaneous ICD (S-ICD). The ICD 106 can communicate with one or more LCPs 102 and/or an ICM. The ICD 106 is shown as including a controller 412, a battery/supply regulator 426, an ECG amplifier and/or filter 414, memory 419, a conductive communication receiver (RX) 442, a conductive communication transmitter (TX) 432, an RF telemetry circuit 464, a shocking circuit 462 and an electrode configuration switch 413. The battery and/or supply regulator 426 provides one or more constant voltages to the various components of the ICD 106. The receiver 442, in this example embodiment, is shown as including a message amplifier and/or filter 440, and a message decoder 438, and is configured to receive conductive communication signals from one or more LCPs (e.g., 102a and/or 102b), and, in some embodiments, an ICM and/or an external device. The controller 412, which is used to control the operation of the ICD 106, can include, e.g., one or more processors (or equivalent control circuitry), RAM and/or ROM memory, logic and timing circuitry, state machine circuitry, and/or I/O circuitry, but is not limited thereto. The controller 412 can also include a clock circuit, or a separate clock circuit (not shown) can provide a clock signal to the controller 412.

The controller 412 is shown as including a timing control module 418, an arrhythmia detector module 420, and an optional arrhythmia discriminator module 422, but can also include alternative and/or additional modules not specifically shown. The timing control module 418 can control the timing of the conductive communication windows, conductive communication pulses, shocking pulses, etc. The timing control module 418 may also be used for the timing of refractory periods, blanking intervals, noise detection windows, evoked response windows, alert intervals, marker channel timing, and so on. The arrhythmia detector 420 can be used to detect arrhythmias, and the optional arrhythmia discriminator 422 can be used to distinguish between different types of arrhythmias and/or to determine whether an arrhythmia detection should be classified as a false positive detection. For example, the arrhythmia detector 420 and/or the arrhythmia discriminator 422 can analyze a patient's heart rates and rhythms and determine, based on this analysis, whether the patient is experiencing fibrillation or impending fibrillation. The arrhythmia detector 420 and/or the arrhythmia discriminator 422 may also diagnose ventricular tachycardias and atrial tachycardias, but are not limited thereto, based on an EGM sensed by the ICD 106 and/or based on messages provided to the ICD 106 by one or more LCPs 102.

In the embodiment shown in FIG. 4, the ICD 106 is shown as being electrically connected to five electrode terminals 425a, 425b, 425c, 425d, and 425e, each of which is electrically connected to a respective electrode. For example, the electrode terminal 425a can be electrically connected to a case electrode (e.g., 125a), which is also known as a cannister or can electrode. The electrode terminals 425b, 425c, 425d, and 425e are electrically connected to electrodes on one or more leads (e.g., 103) that are coupled to the ICD 106. In an example embodiment, the electrode terminal 425b is electrically connected to a distal ring or tip electrode (e.g., 125b), the electrode terminal 425c is electrically connected to a proximal ring electrode (e.g., 125c), the electrode terminal 425d is electrically connected to a distal coil electrode (e.g., 125d) (also known as a parasternal coil electrode), and the electrode terminal 425e is electrically connected to a proximal coil electrode (e.g., 125e) (also known as a traverse coil electrode). The electrode terminals 425a, 425b, 425c, 425d, and 425e, can be collectively referred to as electrode terminals 425, or individually as an electrode terminal 425. The electrodes 125a, 125b, 125c, 125d, and 125e can be collectively referred to as electrodes 125, or individually as an electrode 125. The electrodes 125 (via the electrode terminals 425) are connected to the electrode configuration switch 413, which can also be referred to herein as switches 413. The electrode configuration switch 413, under the control of the controller 412, can connect any two or more of the electrode 125 to the ECG amplifier and/or filter 414, the conductive communication RX 442, the conductive communication TX 432, or the shocking circuit 462. The electrodes 125, as will be described in more detail below, can be used to transmit and receive conductive communication signals to/from one or more LCPs, one or more other types of IMDs, and/or an external device (e.g., a programmer or bedside monitor), and can also be used to sense an electrocardiogram (ECG).

In certain embodiments, the amplifiers and/or filters 414, 440, and 436 are each differential circuits that are intended to be connected to a pair of the electrodes 125 by the switches 413 under the control of the controller 412. For an example, the switches 413 can be controlled to connect any pair of the electrodes 125a, 125b, 125c, 125d, 125e to the message amplifier and/or filter 440. It is also possible that the switches 413 can connect two electrodes 125 directly to one another. For an example, the switches 413 can connect the electrode 125a to a first input of the message amplifier/filter 440, and connect the electrodes 125b and 125c to one another and to a second input of the message amplifier/filter 440. Beneficially, connecting together two or more electrodes (e.g., 125b and 125c) to a same node (e.g., to the same input node of the message amplifier/filter 440) can effectively average or create a virtual vector which is between the two or more electrode locations, which enables sensing of a signal that is effectively an average of the signals detected at the two separate electrodes.

The conductive communication receiver 442, which is shown as including the message amplifier and/or filter 440, and the message decoder 438, is configured to receive conductive communication signals from one or more LCPs (e.g., 102a and/or 102b) or an external device.

The message amplifier and/or filter 440 is configured to amplify and/or filter conductive communication signals received from another IMD or an external device. The amplifier portion can be used to increase the relatively small amplitudes of such conductive communication signals. The filter portion can be a high-pass filter or a bandpass filter adapted to separate an electrocardiogram (ECG) signal from conductive communication signals. The message decoder 438 can be configured to decode conductive communication signals received from another IMD or an external device into a format that the controller 412 can understand. The specific type of decoding performed by the message decoder 438 can depend upon the specific coding of the conductive communication signals received from another IMD or an external device, e.g., on-off keying, frequency-shift keying, frequency modulation, or amplitude shift keying, but not limited thereto. It is also possible that the message decoder 438 be implemented as a comparator that compares the output of the message amplifier and/or filter 440 to a threshold, and outputs binary 1s and 0s based on results of the comparisons, and thereby provides a bitstream to the controller 412. The controller 412 can then perform the specific type of decoding that is dependent on how the conductive communication messages were encoded by the transmitting device.

In certain embodiments, the ICD 106 includes one, two or more additional instances of the conductive communication receiver 442, with example additional instances of the conductive communication receiver labeled 442b and 442c. Each additional instance of the receiver 442 can include its own respective message amplifier and/or filter 440 and its own respective message decoder 438, and can provide its own bitstream to the controller 412. Using the switches 413, which are controlled by the controller 412, each of the different receivers (e.g., 442, 442b, and 442c) can be electrically coupled to a different pair or combination of the external electrodes 125a, 125b, 125c, 125d, and 125e, and can thereby be electrically coupled to a different conductive communication vector.

The conductive communication transmitter 432 is configured to transmit (under the control of the controller 412) conductive communication signals to one or more IMDs implanted within a patient and/or an external device. The conductive communication signals can also be used to program, interrogate, and/or obtain notifications and/or other types of diagnostic information from one or more IMD(s). The transmitter 432, in this example embodiment, is shown as including a message encoder and/or modulator 430 and an amplifier 436. The transmitter 432, because it outputs conductive communication pulses, can also be referred to as a pulse generator.

The conductive communication receiver(s) 442 and the conductive communication transmitter 432 are example components of conductive communication circuitry 452 of the ICD 106. In FIG. 4, the switches 413 are located between the conductive communication circuitry 452 and the electrodes 125a, 125b, 125c, 125d, and 125e that are part of or communicatively coupled to the ICD 106. The switches 413, under the control of the controller 412, enable different conductive communication vectors to be electrically coupled to the conductive communication circuitry 452, or portions thereof. While five electrodes 125 are shown in FIG. 4, it is possible that more or less than five electrodes 125 are part of, or communicatively coupled to, the ICD 106.

The controller 412, which can include one or more processors, and/or the like, can execute operations based on firmware stored in non-volatile memory (Flash). The non-volatile memory can also be used to store parameters or values that are to be maintained when power is removed. The controller 412 can use volatile memory or RAM as general storage for information such as ECG data, status information, swap memory, and other data. Such memory, shown in FIG. 4 as memory 419, can be included within the controller 412 and/or can be communicatively coupled to the controller 412.

The switches 413 can also connect various combinations of the electrodes to an impedance measurement circuit 450. The impedance measurement circuit 450 includes inputs to collect multiple measured impedances between corresponding multiple combinations of electrodes. For example, the impedance measurement circuit 450 may collect a measured impedance for each or a subset of the active sensing vectors. Optionally, the impedance measurement circuit 450 may measure respiration or minute ventilation; measure thoracic impedance for determining shock thresholds: detect when the device has been implanted; measure stroke volume; and/or detect the opening of heart valves, etc.

The shocking circuit 462 generates shocking pulses of low (e.g., up to 0.5 joules), moderate (e.g., 0.5-10 joules), or high energy (e.g., 11 to 80 joules), as selected by the controller 412. Such shocking pulses are applied to a patient's heart through at least two shocking electrodes selected, for example, from the various electrodes 125. The electrodes selected to deliver a shock can be referred to herein as a shocking vector. Example shocking vectors include, but are not limited to, the following: the distal (also referred to as parasternal) coil electrode 125d to the proximal (also referred to as transverse) coil electrode 125e; the distal (also referred to as parasternal) coil electrode 125d and the proximal (also referred to as transverse) coil electrode 125e electrically coupled to one another (to form an anode of the vector) to the case (also known as canister, or can) electrode 125a (that provides the cathode of the vector); the distal ring (or tip) electrode 125b electrically coupled to the distal (also referred to as parasternal) coil electrode 125d (to form an anode of the vector) to the case (also known as canister, or can) electrode 125a (that provides the cathode of the vector); and the distal (also referred to as parasternal) coil electrode 125d to the case (also known as canister, or can) electrode 125a. Other variations are also possible and within the scope of the embodiments described herein.

As noted above, there are situations in which patients are exposed to noisy electromagnetic environments and the conductive telemetry channels 105, 107, 111, 113, and 115 between the LCPs 102a and 102b, the NV-ICD 106, and other devices that may be in the system are interfered with by noise. The noise signals can be misinterpreted by a receiving device as a false i2i signal and processed by device's firmware, causing undefined behavior like change of programming parameters. The signals transmitted from an LCP 102a or LCP 102b to NV-ICD 106 are especially subject to noise interference due to the weaker signal reception on NV-ICD 106 due to its relative alignment and placement with respect to LCP 102a and LCP 102b.

The embodiments described herein are generally described as being used for improving communication between two or more IMDs. However, it is noted that the embodiments described below can also be used to improve communication between an external medical device (EMD) and one or more IMDs. Such an EMD can be an external programmer, a bedside monitor, a remote monitor, or the like. For a specific example, if an EMD attempts to communicate with one or two IMDs, while two IMDs are communicating with one another, there is a potential risk that a data frame being transmitted by the EMD can clash with a data frame being transmitted between the IMDs. The embodiments described herein can reduce the risk of data frames clashing in this situation. This is just one example way of using an embodiment described herein to improve communication between an EMD and one or more IMDs, which example is not meant to be limiting. More generally, embodiments described herein can be used to improve communication between two or more devices, at least one of which is an IMD. In the following discussion, when at least two devices are described as communicating with one another, it is assumed that at least one of the devices comprises an IMD. While it is possible and likely that all of the devices are IMDs, it is also possible that at least one of the devices is an EMD.

The following discussion presents a conductive telemetry communication system between LCP 102a, 102b, and NV-ICD 106 that can be compatible with currently employed LCP to LCP communication systems. The communication link from NV-ICD 106 to LCP 102a or 102b can use the same information encoding/decoding method of the existing LCP implant-to-implant communication systems, whereas the communication link from an LCP to the NV-ICD uses embodiments of an information encoding/decoding method that has enhanced performance of noise resistance. This newly introduced information encoding/decoding method can be implemented with minimal change on the currently existing LCP firmware, and no hardware change is necessary on the LCPs 102a and 102b. The techniques can also apply to multiple LCP communications if hardware and firmware changes are made. This same techniques can also apply to other implant-to-implant communications with two or more implanted medical devices (IMDs).

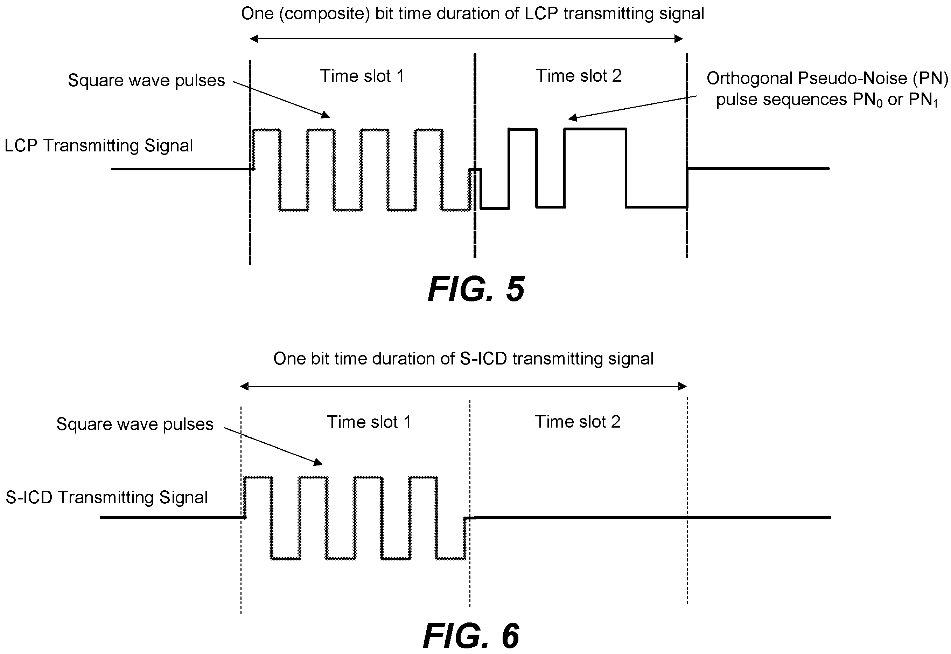

More specifically, the following embodiments present examples of an implant-to-implant communication system between LCP 102a or 102b and NV-ICD 106 that is compatible with the currently available i2i communication system between the LCPs 102a and 102b. In embodiments of this communication system, each one bit of LCP transmitting signals are encoded in two consecutive time slots, as illustrated in FIG. 5.

FIG. 5 illustrates an example of a transmitting signal waveform that includes one bit to be communicated from one LCP to another LCP (in a time slot 1) and that also includes a further one bit to be communicated to an NV-ICD (in a time slot 2), which are combined to form a composite bit. More generally, FIG. 5 illustrates an example of a transmitting signal waveform including one bit to be sent from a first implantable medical device (IMD1) to a second IMD (IMD2) (in a time slot 1) and also including a further one bit to be sent from the IMD1 to a third IMD (IMD3) (in a time slot 2) that are combined to form a composite bit. As used herein, a composite bit is a bit of a signal with multiple, i.e., at least two, time slots, each of which carries a bit of a signal for a different device. The embodiment of FIG. 5 includes two time slots (respectively for IMD2 and IMD3), but other embodiments can include additional time slots to accommodate communication with additional implantable medical devices. Depending on the embodiment, the assignment of devices to time slots 1 and 2 can be in either order and additional time slots could be added to the composite bit for additional device communications. In the embodiment of FIG. 5, for the composite bit the signal waveform in time slot 1 can be compatible with certain existing conductive i2i communication systems and can be decoded by the receiving LCP (or more generally, the IMD2), either as a set of square wave pulses (a "bit 1" in this embodiment) or as a flat waveform (a "bit 0" in this embodiment). FIG. 5 illustrates a "bit 1" in time slot 1. (In an alternative embodiment, square wave pulses within the time slot 1 can represent a "bit 0" and a flat waveform within the time slot 1 can represent a "bit 0.") The signal waveform in time slot 2 are encoded with one of two orthogonal Pseudo Noise (PN) pulse sequences $PN_0$ and $PN_1$ as "bit 0" and "bit 1" correspondingly. The waveform in time slot 2 will be decoded by the receiving NV-ICD 106 (or more generally, the IMD3). The terms "bit 0" and "bit value 0" are used interchangeably herein, and the terms "bit 1" and "bit value 1" are used interchangeably herein. Encoding a bit value "1" and a bit value "0" with respective PN pulse sequences provides an enhanced noise resistance feature for the receiving NV-ICD 106 (or more generally, the receiving IMD3). The decoding procedure at both a receiving LCP (e.g., 102b, for a transmitting LCP 102a) and a receiving NV-ICD 106 will be synchronized with the transmitting LCP's wake up signal pulse. More generally, the decoding procedure at both the receiving IMD2 and the receiving IMD3 will be synchronized with the transmitting IMD1's wake up signal pulse, as will be described in more detail below. Note that the pseudo noise (PN) waveforms are not being used to encode or modulate another signal, but are themselves the "1" and "0" bit values.

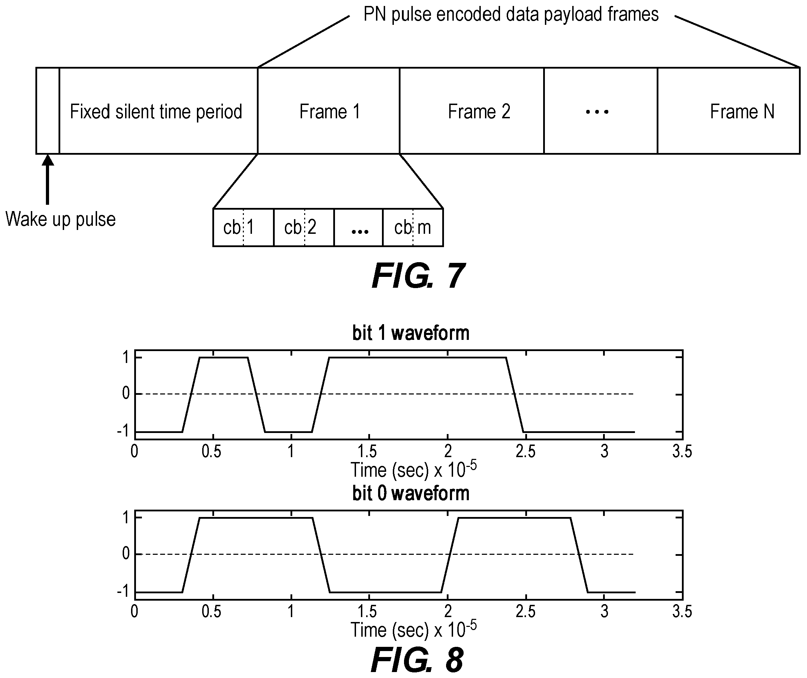

The composite bits of a message can be assembled into a series frames, where the number of composite bits per frame can be a fixed number, or can vary depending on the embodiment. In certain embodiments, a wake-up pulse signal is followed by a series of payload frames for implant-to-implant communication, and each payload frame includes multiple composite bits, including data stream encoded by $\{PN_0, PN_1\}$ pulse sequences in one of the time slots. An embodiment for the format layout of such data frames is shown in FIG. 7, which is discussed further below.

FIG. 6 illustrates an example of a transmitting signal waveform (one bit) from an NV-ICD to an LCP, or more generally, from the IMD3 to the IMD1 or the IMD2. Each one bit of the NV-ICD 106 (or more generally, IMD3) transmitting signals can be the same as the signals available in an existing i2i communication system for the receiving LCP 102a and/or 102b (or more generally, for the receiving IMD1 and/or IMD2). For example, as shown in FIG. 6, time slot 1 is used for the data value to be transmitted, where, as in FIG. 5, this can either be a sequence of square wave pulses (again a "1" bit value in this example) or a flat waveform (a "0" bit value in this example), wherein the sequence of square wave pulses can also be referred to herein as a square wave pulses waveform. In other words, the time slot 1 includes a waveform (e.g., a square wave pulses waveform, or a flat waveform) corresponding to a data value. Time slot 2 is used by the receiving LCP(s) 102a and/or 102b to determine whether a "0" or "1" was detected during time slot 1. In other words, the receiving IMD(s) perform decoding during the time slot 2. In each of the FIGs. showing example signal waveforms, such as FIGs. 5 and 6, the vertical axis represents voltage and the horizontal axis represents time, unless stated otherwise.

FIG. 7 illustrates an embodiment for a pseudo noise (PN) pulse encoded data frame format layout, where each frame includes a specified number m of composite bits of the form of either FIG. 5 or FIG. 6. The specified number m can be fixed, or can be programmable, depending upon the specific implementation. In this embodiment, the signal begins with a wake up pulse, followed by a specified silent time period after which begins a specified number N of frames, Frames 1 to N, each having a number (the specified number m) of composite bits formatted as in FIG. 5 or FIG. 6, depending on the on the communication direction. The specified silent time period can be fixed, or can be programmable, depending upon the specific implementation. The specified number N of frames can be fixed, or can be programmable, depending upon the specific implantation. As illustrated for Frame 1, each frame is made up of a specified number m of composite bits, cb 1 to cb m, that each have a first time slot and a second time slot, as represented by the broken line in each composite bit (cb). As noted above, the specified number m can be fixed, or can be programmable, depending upon the specific implementation. These techniques can help to mitigate false positive detections of i2i messages that may disrupt i2i communication between IMDs in noisy signal environments.

Considering the Pseudo Noise (PN) pulse sequences further, these can be used for encoding bit values of "0" and "1" for the communication link from the LCP 102a or 102b to the NV-ICD 106 (and more generally, from IMD1 or IMD2, to IMD3) as illustrated in FIG. 5. Generally, each PN pulse sequence includes a sequence of time-wise orthogonal pulses. More specifically, each PN pulse sequence includes a sequence of pulses whose integral over the duration of the sequence of pulses is equal to zero. In the embodiments presented here, the PN pulse sequences have the following features (i.e., meet the following pseudo noise conditions):

1) The time integral of each of the PN sequences, PN0 and PN1, over its PN sequence duration should be zero:

$$\int PN_0\,(t)dt = 0$$

$$\int PN_1\,(t)dt = 0$$

2) The inner product of the $PN_0$ and $PN_1$ pulse sequences over the PN sequence duration should be zero, i.e., the $PN_0$ and $PN_1$ pulse sequences are orthogonal to one another:

$$\int PN_0\,(t) \cdot PN_1(t)dt = 0$$

3) The inner product of each of the $PN_0$ or $PN_1$ pulse sequences, with itself, over the PN sequence duration should be non-zero:

$$\int PN_0\,(t) \cdot PN_0(t)dt \neq 0$$

$$\int PN_1\,(t) \cdot PN_1(t)dt \neq 0$$

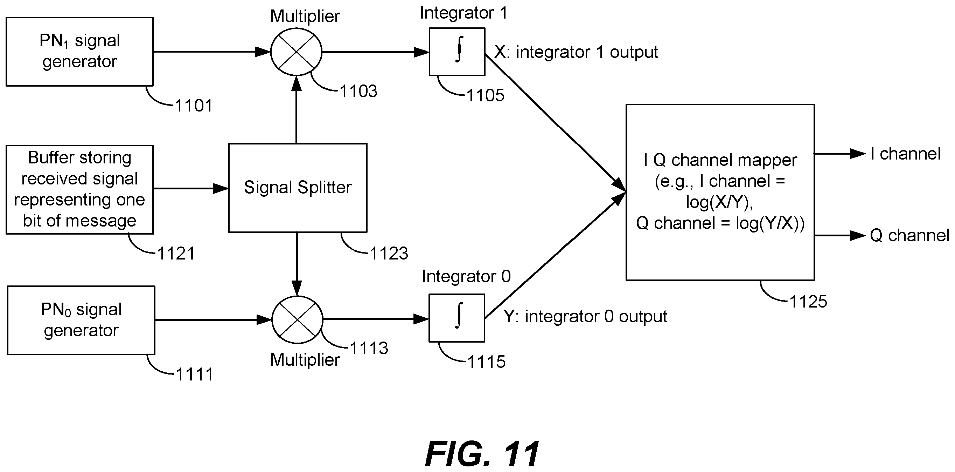

The requirement that the inner product of the $PN_0$ and $PN_1$ pulse sequences over the PN sequence duration should be zero effectively reduces the random noise during the decoding and distinguishes the bit value "1" and the bit value "0" waveforms. The requirement that the inner product of each of the PN0 or PN1 pulse sequences, with itself, over the PN sequence duration should be non-zero allows for the decoding described below with reference to FIG. 11.

FIG. 8 illustrates an example of one embodiment of waveforms for a pair of pseudo noise pulse sequences $\{PN_0, PN_1\}$ that meet the pseudo noise conditions given above. Both the $PN_1$ waveform at top in FIG. 8 and the $PN_0$ waveform at bottom in FIG. 8 average to zero over the time slot interval (i.e., the time integral of each of the PN sequences over its pulse duration equals zero) and, as can be seen by comparing when $PN_1$ and $PN_0$ are high and low, they are orthogonal to one another over the interval. Alternate embodiments can also use pseudo noise in the time slot 1 for LCP to LCP (or other i2i) communications, in which case an additional pair of pseudo noise pulse sequences $\{PN_0', PN_1'\}$ can be used, where all four pseudo noise pulse sequences are mutually orthogonal.

FIG. 9 is a block diagram of an embodiment for encoding of two example series of bits to be combined into composite bits as illustrated FIG. 5 for transmission. At block 901 a sequence of bits to be transmitted from LCP 102a to LCP 102b (or more generally, from IMD1 to IMD2) can be generated by the controller 112 of the LCP 102a (or more generally, of the IMD1) and are encoded at block 903 (e.g., using a look up table (LUT), but not limited thereto) into either a series of pulses ("p") for a "1" bit value or a flat waveform ("f") for a "0" bit value. For example, the series of transmitting bits [0, 1, 1, 1] become the transmitting pulses series in block 905 of [f, p, p, p]. In an embodiment, for the sequence of transmitting bits in block 911 to be transmitted from LCP 102a to NV-ICD 106 (or more generally, from IMD1 to IMD3), a lookup table LUT at block 913 can be used to determine the corresponding set of transmitting pulses in block 915. For example, the sequence of transmitting bits [0, 1, 0, 1] at block 911 is encoded to the PN sequences [$PN_0$, $PN_1$, $PN_0$, $PN_1$] at block 915. In certain embodiments, the pseudo random noise pulse sequences can be predefined and hard coded in the transmitting LCP's (or more generally, IMD's) firmware as a lookup table (LUT). For each bit value (either a bit value "1" or "0"), the LCP firmware can then fetch the corresponding PN pulse sequence of $\{PN_1, \text{ or } PN_0\}$ from the lookup table at block 913 and encode the bit waveform in time slot 2 of FIG. 5. The composite bits are then assembled at block 921 as illustrated in FIG. 5. For the example, at block 921, the composite bits (corresponding to the bits of blocks 901 and 911), can be [$f/PN_0$, $p/PN_1$, $p/PN_0$, $p/PN_1$], where the "/" is used to separate the time slots of a composite bit. The pulses of the composite bits can then be assembled into the data frame format of FIG. 7 (for transmission) by the pulse generator 116 of LCP 102a. Other embodiments can reverse which device is assigned to time slots 1 and 2. If both time slots use pseudo noise encoding, then encoding block 903 can also be or utilize a lookup table (LUT) similar to the LUT used at block 913, with a second pair of mutually orthogonal pseudo noise pulse sequences.

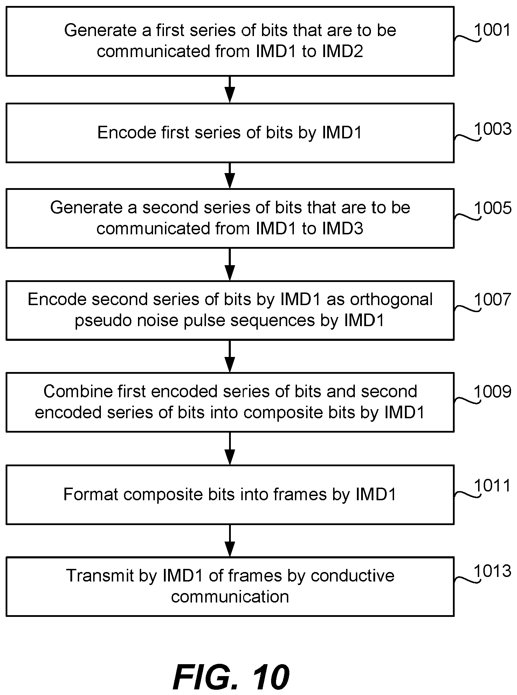

FIG. 10 is a flowchart of an embodiment for the encoding of signals for implant-to-implant communication from a first implantable medical device (IMD1) to a pair of other implantable medical devices (IMD2 and IMD3) using the format(s) of FIGs. 5-9. Beginning at step 1001, the transmitting device IMD1 generates a first series of bits that are to be communicated to IMD2. In this example IMD1 and IMD2 are respectively LCP 102a and LCP 102b, and the steps performed by IMD1 can be performed by the controller 112 and the pulse generator 116 of LCP 102a. The first series of bits to be transmitted are then encoded at step 1003 into time slots 1 of composite bits, in accordance with the embodiment of FIG. 5, as either a series of square wave pulses and/or flat waveforms. Steps 1001 and 1003 correspond to blocks 901, 903, 905 in FIG. 9. At step 1005, the transmitting device IMD1 generates a second series of bits that are to be communicated to IMD3, where in this example IMD3 is NV-ICD 106. The second series of bits to be transmitted are then encoded at step 1007 to the pair of orthogonal pseudo noise (PN) waveforms, where steps 1005 and 1007 correspond to blocks 911, 913, 915 in FIG. 9, and the pseudo noise encoding can be performed using a lookup table (LUT), e.g., as described with respect to LUT used at block 913. It is noted that the order that steps 1001, 1003, 1005, and 1007 shown in FIG. 10 can be changed, so long as step 1001 is performed before step 1003, and step 1005 is performed before step 1007. For example, it is possible that step 1005 is performed at substantially the same time as step 1001, or before step 1001. For another example, it is possible that step 1007 is performed at substantially the same time as step 1003, or before step 1003.

Still referring to FIG. 10, at step 1009 IMD1 can combine the two series of transmitting pulses (that have been encoded) into the composite bit format of FIG. 5, corresponding to the composite bits 921 of FIG. 9. In step 1011 the composite bits (formatted as described with reference to FIG. 5) can then be formatted by IMD1 into the frame format of FIG. 7. The formatted frames are then transmitted using conductive communication at step 1013.

Turning now to demodulating the pseudo noise pulse sequence at the receiving device, FIG. 11 is diagram for an embodiment for a pseudo noise pulse demodulation system. For example, in the NV-ICD embodiment of FIG. 4, these elements can be part of the conductive communication RX 442 and, more specifically, part of the message decoder 438. The pseudo noise pulse sequence demodulation at NV-ICD 106 (or more generally, IMD3) is done by making use of the orthogonality of $PN_0$ and $PN_1$. The individual bits of the received signal can be buffered at a buffer 1121 and supplied to a signal splitter 1123 that supplies the received signal to the multipliers 1103 and 1113. The NV-ICD 106 receiver will also know the pair of pseudo noise pulse signals and can provide the $PN_1$ signal from a signal generator 1101 and the $PN_0$ signal from a further signal generator 1111 to respective multipliers 1103 and 1113. Respective integrators 1105 and 1115 then integrate the output of multipliers 1103 and 1113 over the duration of time slot 2. Due to the orthogonality of $PN_1$ and $PN_0$ (and ignoring noise), if the received bit is $PN_1$ the output X of integrator 1105 will be 1 and the output Y of integrator 1115 will be 0. Conversely, if the received bit is $PN_0$ then the outputs will be X=0 and Y=1. The X and Y values are then converted into, for instance, in-phase (I)/quadrature (Q) format at an I-Q channel mapper 1125. A number of mappings can be used to map onto the I and Q channels, such as the example embodiment of I channel = log(X/Y) and Q channel = log(Y/X). This set of log based I-Q channel mappings is just one of the implementations for I-Q channel constellation mapping. Other mapping algorithms based on other functions can also be considered based on actual device hardware features, while being within the scope of the embodiments described herein. If multiple pairs of mutually orthogonal pseudo noise pulse waveforms are used, such as when time slot 1 is also pseudo noise encoded or in the multiple device communication embodiments discussed below, different mappings can be used for each pair.

In terms of the circuitry for implementing the pulse demodulation system of FIG. 11, a number of different embodiments are possible. For example, the I-Q channel mapper 1125 can have a digital implementation, and the elements to its left in FIG. 11 (e.g., elements 1101, 1103, 1104, 1111, 1113, 1115, 1121 and 1123) can be implemented as analog circuits. An analog to digital conversion can be done by introducing an ADC or ADCs (not shown) between the integrators 1105 and 1115 and the I-Q mapper 1125, or at other points in system. For example, the integrators 1105, 1115 could work in the digital domain following an ADC after each of multipliers 1103, 1113. Other variations are also possible and within the scope of the embodiments described herein.

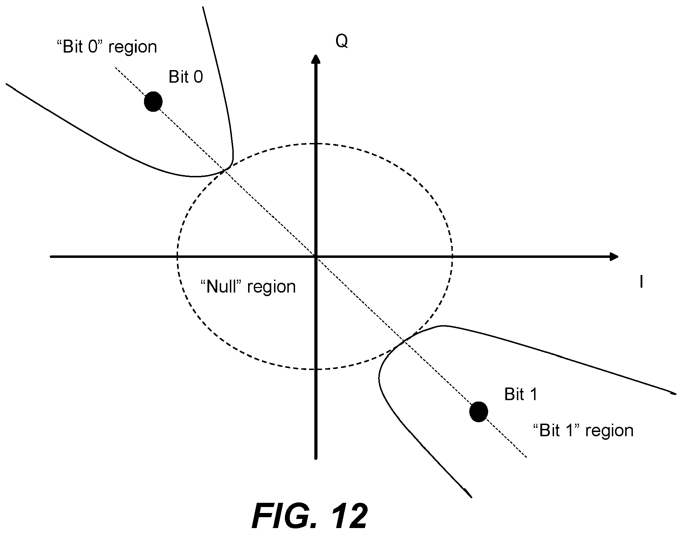

FIG. 12 illustrates an embodiment of a decoding constellation diagram for the I-Q channel in FIG. 11 at the receiving NV-ICD (or more generally, at the IMD3). The demodulated (I, Q) value pairs are decoded as a "0" bit value if they fall within the "Bit 0" region, and as "1" bit value if they fall within the "Bit 1" region. The (I, Q) value pairs are decoded as a "null" bit if they fall into "Null" region. The "Null" means neither "0" nor "1" is reliably decoded. If a "Null" is decoded in a message, then the whole message can be regarded as a corrupted and discarded. In this case, a noise search count in the receiving IMD (e.g., NV-ICD) firmware can be increased by one to prepare for noise reversion events. As the receiving signal to noise ratio (SNR) deteriorates, both the resultant "Bit 0" and "Bit 1" regions can be expanded into the "Null" region to increase the range of acceptable "Bit 0" and "Bit 1". This decoding mechanism can help reduce the likelihood of a falsely decoded implant-to-implant message to be processed by the receiving IMD (e.g., NV-ICD) as the noise-contaminated bits are disregarded as "null" bits instead of false positive bits with opposite polarity. For example, while decoding a string of received bits, if a result falls into the "Null" region, this decoded value will be of low confidence and the data frame can be discarded as corrupted and an error signal returned to the transmitting device, which may cause the transmitting device to retransmit the message. This decoding system also has improved performance for low receiving SNR values, as discussed further below.

In the transmitting signal of NV-ICD 106 for the embodiment of FIG. 6, this signal can be transmitted by conductive communication as a broadcast signal receivable by either LCP 102a or LCP 102b. When there are more than two implantable medical devices (IMDs), it may be beneficial to build a private session between each individual pair of devices such that the communication between a pair of IMD should not interfere with other IMDs within telemetry reception range. The next few figures consider embodiments for implementing such private sessions.

More specifically, these embodiments use more than one pair of $PN_0$ and $PN_1$ for encoding bit 1 and bit 0 values, where each of these $\{PN_0, PN_1\}$ pairs are also orthogonal to the other $\{PN_0, PN_1\}$ pairs. All the available

mutually orthogonal $\{PN_0, PN_1\}$ pair options can be stored in device firmware and assigned with a unique pair ID. It is possible to use multiple $\{PN_0, PN_1\}$ pairs to implement private sessions between two devices without sharing the communicated information with other implanted devices. Two methods to build private sessions with multiple $\{PN_0, PN_1\}$ pairs are presented: a Public-Private $\{PN_0, PN_1\}$ pair method; and a Private $\{PN_0, PN_1\}$ pair method.

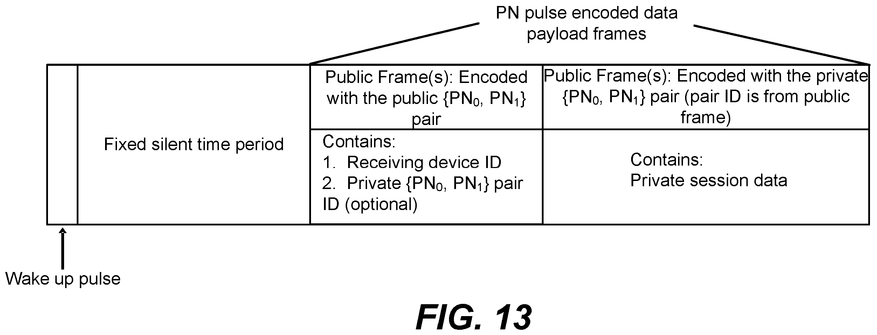

FIG. 13 illustrates an embodiment for the data frame format of the Public-Private $\{PN_0, PN_1\}$ pair method. As in FIG. 7, the frame format again begins with a wake up pulse followed by a specified silent time period. Following the silent time period are one or more data payload frames that each have two parts, the "Public Frame" and the "Private Frame." In the example embodiment, there is one public frame and one private frame, each of a specified number of bits, but other embodiments can have additional public or private frames. The public frame is encoded by a public $\{PN_0, PN_1\}$ pair that is shared with every IMD (e.g., LCP or NV-ICD). Each IMD is programmed to use the public $\{PN_0, PN_1\}$ pair to decode the payload of the public frame time slot. The public frame includes the intended receiving device's ID, or devices' IDs if multiple receiving device are to use the same channel in a broadcast manner, and the private $\{PN_0, PN_1\}$ pair ID that the transmitting device will use to encode the private frame, allowing for the transmitting device to dynamically assign private $\{PN_0, PN_1\}$ pairs to other devices. Note that including the private $\{PN_0, PN_1\}$ pair ID in the public frame can be optional if each device is pre-configured with a private $\{PN_0, PN_1\}$ pair. After the transmitting device finishes sending the public frame, it will switch to the private $\{PN_0, PN_1\}$ pair to encode and transmit the private frame. The intended receiving device will also switch to private $\{PN_0, PN_1\}$ pair to decode the upcoming private frame data and for communicating back to the first device. Consequently, once the private $\{PN_0, PN_1\}$ pair is established between two devices, a public frame need not be included in subsequent exchanges.

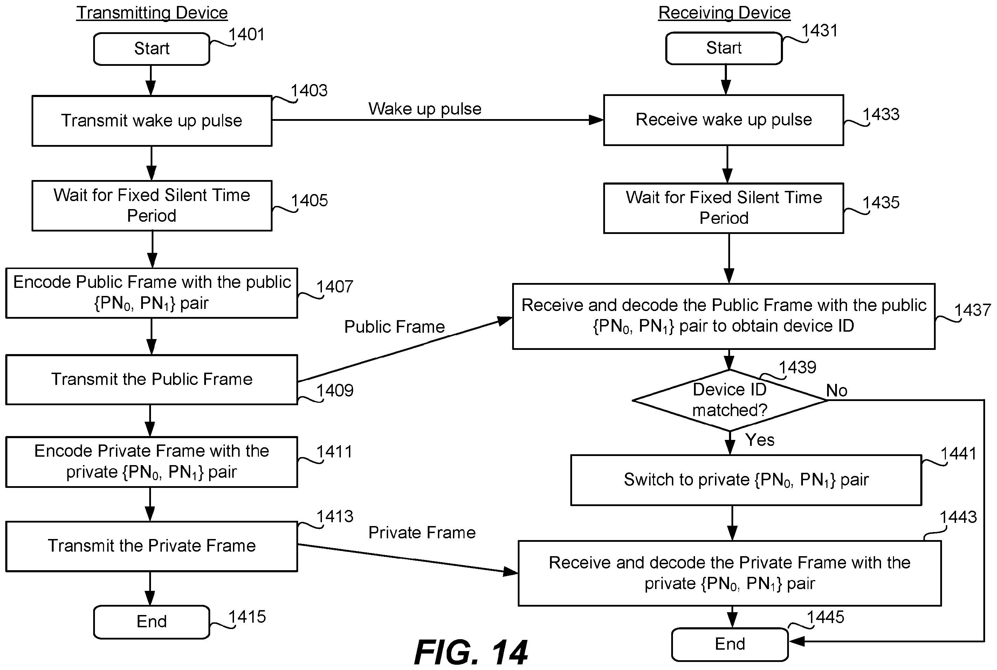

FIG. 14 is a flowchart of an embodiment for the public-private $\{PN_0, PN_1\}$ pair technique. In FIG. 14, the steps for the transmitting device are shown at the left and the steps for the receiving device are shown at the right. On the transmitting side, the flow starts at 1401 with a wake up pulse transmitted at step 1403, after which the transmitting device waits for the specified silent time period at step 1405. The specified silent period can be fixed, or can be programmable, depending upon the specific implementation. On the receiving side, the flow starts at 1431 with the wake up pulse received at step 1433, after which the receiving device waits for the specified silent time period at step 1435. After waiting for the specified silent time period, at step 1407 the transmitting device encodes the public frame that specifies the receiving device's ID and, optionally, the private $\{PN_0, PN_1\}$ pair, that is then transmitted at step 1409. In an embodiment, the encoding that is performed at step 1407 occurs during the waiting at step 1405, so that the public frame can be transmitted at step 1409 as soon as the silent time period expires.

On the receiving device, the public frame is received and decoded at step 1437, which allows any receiving device to obtain the ID of the intended receiving device or devices. Step 1439 checks whether a receiving device is the intended receiving device (i.e., the intended recipient) based on whether the decoded device ID matches that of the receiving device. If not, the flow on the receiver side ends at step 1445 for any unintended implantable device receiving the public signal. If the ID received and decoded at step 1439 matches that of the receiving device, the receiver switches to the private $\{PN_0, PN_1\}$ pair for subsequent communications. In a dynamic embodiment, the private frame will specify the private $\{PN_0, PN_1\}$ pair that is then saved by a specified receiver to use for decoding the following private frame of the initial communication and any subsequent communications.

On the transmitting side, the private session data is encoded in the private frame with the private $\{PN_0, PN_1\}$ pair at step 1411 and transmitted at step 1413. On the receiving side the private frame is received and decoded using the private $\{PN_0, PN_1\}$ pair at step 1443. The transmission and reception of the data frame then ends at respective steps 1415 and 1445 for the transmitting and receiving devices. Subsequent interchanges between the devices can continue using the established private $\{PN_0, PN_1\}$ pair. It is noted that the encoding in step 1411 can be performed at least partly simultaneously with steps 1407 and/or 1409. Further, it is noted that steps 1411 and 1407 can be interleaved or reversed, assuming the receiving IMD has a sufficiently sized buffer to stored received waveform data. More generally, the order of the various steps can be modified while still being within the scope of the embodiments described herein.

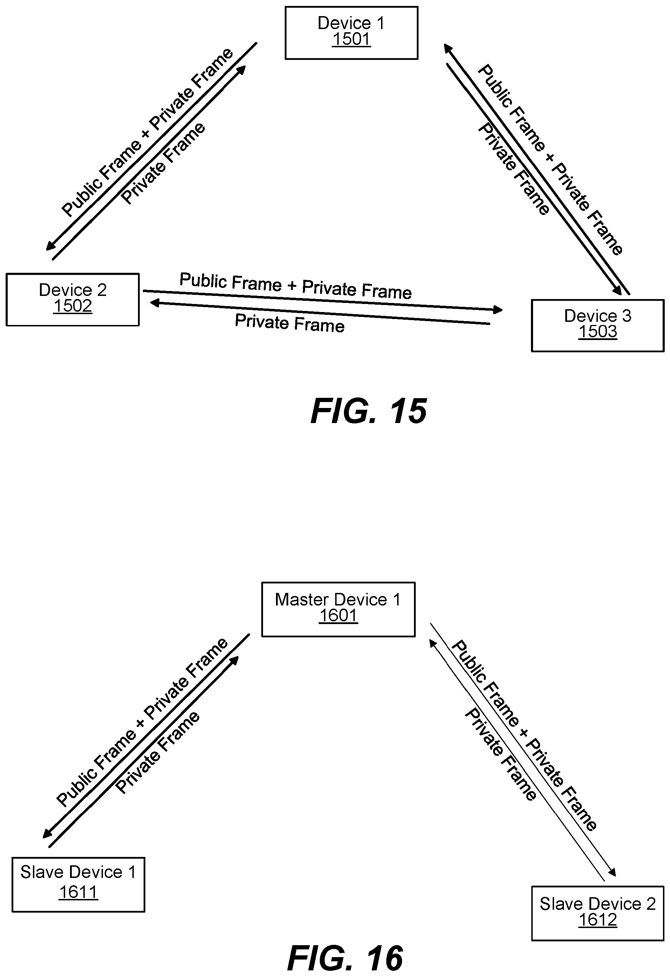

The public-private $\{PN_0, PN_1\}$ pair technique illustrated with respect to FIGs. 13 and 14 can be used to implement a decentralized implant-to-implant communication network among implanted devices, as any device can initiate the private communication session with any intended receiving device with the workflow shown in FIG. 15. This method can also be used to implement a Master/Slave implant-to-implant communication network as well, in which only the master device can transmit the public frame and decide with which slave device to communicate, as illustrated in FIG. 16. In terms of implementation on the devices, on the transmitting side this can be as described above with respect FIGs. 9 and 10, and on the receiving side as described above with respect to FIGs. 11 and 12. A lookup table can again be used for the pseudo noise encoding, but incorporating the mutually orthogonal public and private pulse pairs, where, when dynamically assigning the private pair, the receiving device can store the received private $\{PN_0,$

$PN_1$} pair in memory for later use.

FIG. 15 illustrates an embodiment for a decentralized implant-to-implant communication network topology for using a public-private {$PN_0$, $PN_1$} pair method for a three device embodiment of Device 1 1501, Device 2 1502, and Device 3 1503 as in FIG. 1, although more devices can be included. In the decentralized topology, any of the devices can instigate a private channel with any of the other devices through use of public-private pair arrangement. As shown in FIG. 15, Device 1 1501 can transmit a (public frame + private frame) specifying Device 2 1502, after which communications between Device 1 1501 and Device 2 1502 can be encoded with the private {$PN_0$, $PN_1$} pair. Although not represented in FIG. 15, Device 2 1502 could similarly instigate the communication channel by initially conductively communicating the (public frame + private frame) specifying Device 1 1501. Device 2 1502 can similarly instigate a communication channel with Device 3 1503 and (although not shown) vice versa, and Device 3 1503 can instigate a communication channel with Device 1 1501 and (although not shown) vice versa.

FIG. 16 illustrates an embodiment for a master/slave implant-to-implant communication network using a public-private {$PN_0$, $PN_1$} pair arrangement. Referring back to the example of FIG. 1, depending on the embodiment any of LCP 102a, LCP 102b, or NV-ICD 106 can be the Master Device 1601, although the larger size of ICD 106 can make it be better adapted to be the master in some embodiments. In the master/slave arrangement, the master device 1 1601 transmits the (public frame + private frame) to Slave Device 1 1611 or Slave Device 1612 to establish the private {$PN_0$, $PN_1$} pair for the subsequent conductive communication using the private {$PN_0$, $PN_1$} pair between the device pairs.

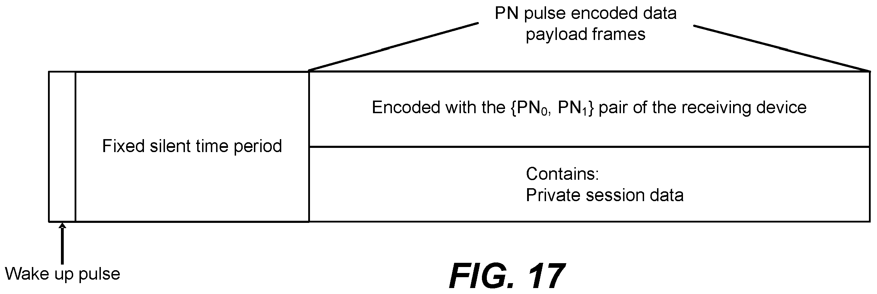

FIG. 17 illustrates an embodiment for the data frame format in a Private {$PN_0$, $PN_1$} pair method. In these embodiments, each device of an implanted system contains a look-up table of the private {$PN_0$, $PN_1$} pairs for each device in the implanted system. The transmitting device will encode the data payload frames with the private {$PN_0$, $PN_1$} pair of the intended receiving device or devices. The receiving device will decode the private session data with the corresponding private {$PN_0$, $PN_1$} pair. As in FIGs. 7 and 13, the frame format again begins with a wake up pulse followed by a first silent time period. The data payload frame or frames will contain the private session data encoded with the {$PN_0$, $PN_1$} pair of the intended receiving device. In accordance with certain embodiments, a group of IMDs can share a private {$PN_0$, $PN_1$} pair. For example, LCPs 102a, 102b could both use the same private pair, whereas the NV-ICD 106 uses another private pair.

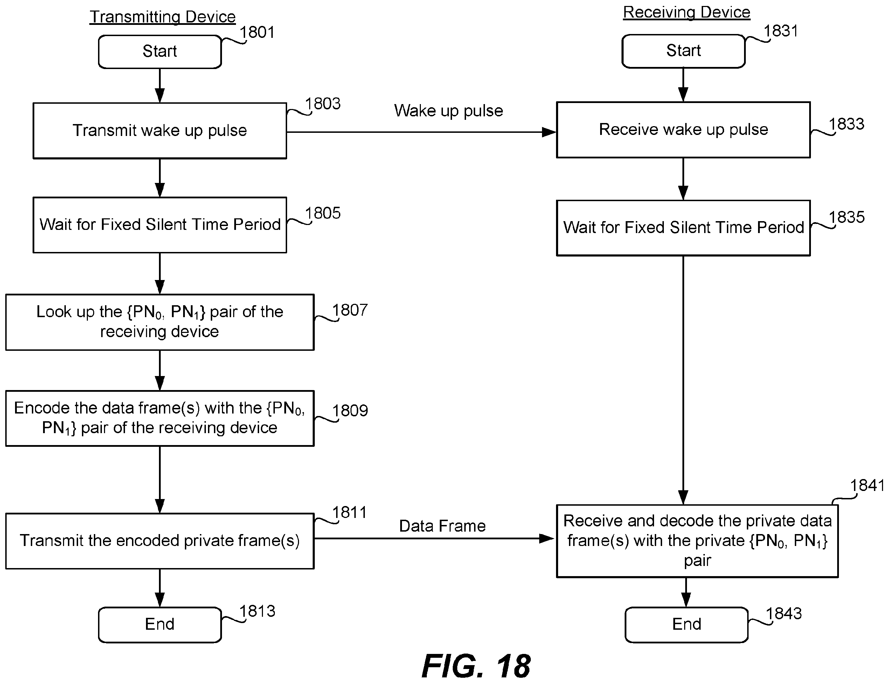

FIG. 18 is a flowchart of an embodiment for the private {$PN_0$, $PN_1$} pair technique. In FIG. 18, the steps for the transmitting device are as shown at the left and the steps for the receiving device are shown at the right. On the transmitting side, the flow starts at 1801 with a wake up pulse transmitted at step 1803, after which the transmitting device waits for the specified silent time period at step 1805. On the receiving side, the flow starts at 1831 with the wake up pulse received at step 1833, after which the receiving device waits for the specified silent time period at step 1835. After waiting the specified silent time period, at step 1807 the transmitting device looks up the {$PN_0$, $PN_1$} pair of an intended receiving device (or devices) and, at step 1809, encodes the data frame or frames with the {$PN_0$, $PN_1$} pair of the intended receiving device or devices.

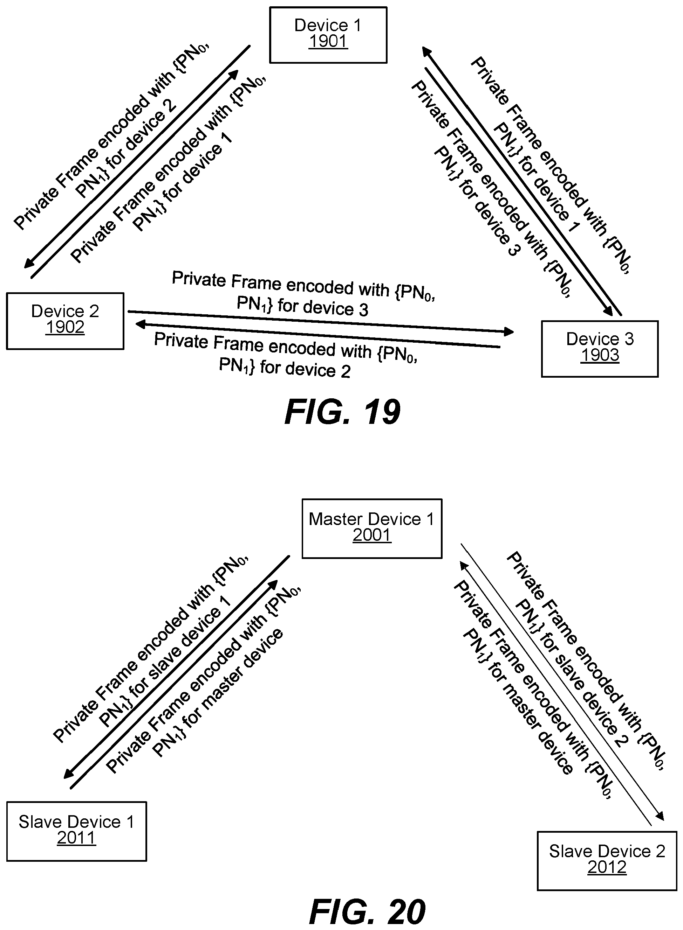

After the silent time period expires, at step 1811 the encoded private frame or frames are transmitted at step 1811, after which the flow for the transmitting device ends at 1813. In an embodiment, the looking up that is performed at step 1807 and the encoding that is performed at step 1809 occur during the waiting at step 1805, so that the private frame can be transmitted at step 1811 as soon as the silent time period expires. In one set of embodiments, a frame can have a specified number of bits. The private frames are received and decoded by an intended receiving device using the device's private {$PN_0$, $PN_1$} pair at step 1841, after which the flow for the receiving device ends at step 1843. Due to the mutually orthogonality of the different {$PN_0$, $PN_1$} pairs, an unintended receiving device will, if it tries to decode the private frames, find the bits of the frame will provide a null output for both the "1" and "0" channel when demodulated. In this way the method of FIGs. 19 and 20 can be used to implement a decentralized implant-to-implant communication network if each device has the complete {$PN_0$, $PN_1$} pair lookup table of other devices. As with the Public-Private arrangement of FIGs. 13-16, the Private {$PN_0$, $PN_1$} Pair method can be implemented as both a decentralized implant-to-implant communication network and a Master/Slave implant-to-implant communication network.

FIG. 19 illustrates an embodiment for a decentralized implant-to-implant communication network using a private {$PN_0$, $PN_1$} pair method for a three device embodiment (as in FIG. 1) that includes Device 1 1901, Device 2 1902, and Device 3 1903, although more devices can be included. Each of the devices encodes the private session data for the intended device or devices with the corresponding private {$PN_0$, $PN_1$} pair in a symmetric arrangement.

FIG. 20 illustrates an embodiment for a master/slave implant-to-implant communication network with public-private {$PN_0$, $PN_1$} pair arrangement. In the master/slave arrangement, only the master device need have the complete {$PN_0$, $PN_1$} pair lookup table of slave devices and each of the slave device needs to have the master device {$PN_0$, $PN_1$} pair.

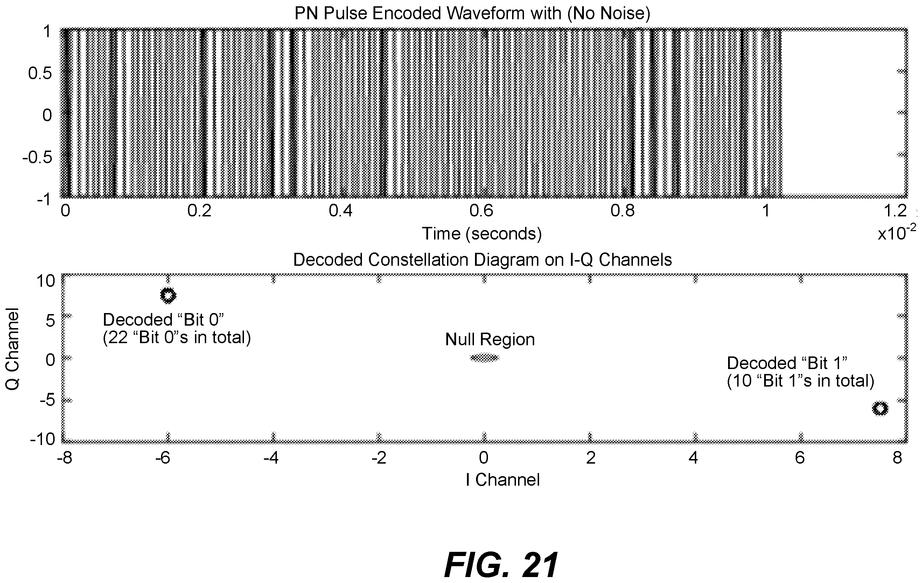

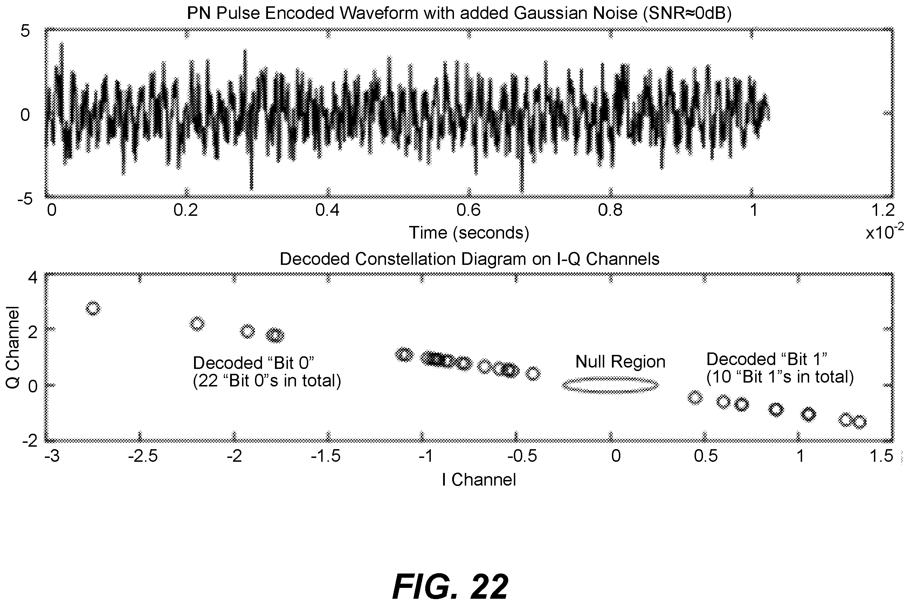

For any of the embodiments presented above, the use of pseudo noise encoding of the data signal can improve the ability of a receiving device to decode the conductively communicated data content. This can be illustrated with respect to FIGs. 21 and 22.

FIGs. 21 and 22 respectively illustrate pseudo noise encoding/decoding without noise and with included additive Gaussian noise. At top of each of FIGs. 21 and 22 is the pseudo noise encoded waveform respectively with no noise and with the added Gausian noise. The corresponding decoded constellation diagram on the I-Q channels is shown underneath for both figures. In the simulations of FIGs. 21 and 22, the pseudo noise pulse sequences shown in FIG. 8 are used for encoding "bit 1" and "bit 0" values for a random sequence of bit values, where the result is shown at top of FIG. 21 for a pseudo noise pulse end encoded waveform is without noise and present well defined values. At the top of FIG. 22 Gaussian noise is added with a Signal to Noise Ratio (SNR) of 0 dB so the values are much less well defined. The two waveforms are then the respective input of the decoding system of FIGs. 11 and 12 as shown at the bottom of FIGs. 21 and 22. In the bottom graph of both FIGs. 21 and 22, the condition of the null region defined as by the acceptable ratio $max\{X/Y, Y/X\}$ as per FIG. 12 is less than 1.5. In both FIGs. 21 and 22, the two versions of PN pulse encoded waveforms is shown at top and the corresponding decoded constellation diagram on I-Q channel is shown underneath in both cases. In FIG. 21 the "bit 0" and "bit 1" values are well separated from each other and the null region. At the bottom in FIG.22, the "bit 0" and "bit 1" decoded value distributions are less well defined, but still allows for the system to successfully decode the waveforms.

The above embodiments were generally described as being used for improving communication between two or more IMDs. However, it is noted that the above embodiments can also be used to improve communication between an external medical device (EMD) and one or more IMDs. For example, it would also be useful for an EMD to use pseudo noise pulse sequences when the EMD communicates with multiple IMDs. Such an EMD can be an external programmer, a bedside monitor, a remote monitor, or the like. For a specific example, if an EMD attempts to communicate with one or two IMDs, while two IMDs are communicating with one another, there is a potential risk that a data frame being transmitted by the EMD can clash with a data frame being transmitted between the IMDs. Using two different pairs of pseudo noise pulse sequences can reduce the risk of data frames clashing in this situation. This is just one example way of using an embodiment described herein to improve communication between an EMD and one or more IMDs, which example is not meant to be limiting. More generally, embodiments described herein can be used to improve communication between two or more devices, at least one of which is an IMD.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

Embodiments have been described above with the aid of functional building blocks illustrating the performance of specified functions and relationships thereof. The boundaries of these functional building blocks have often been defined herein for the convenience of the description. Alternate boundaries can be defined so long as the specified functions and relationships thereof are appropriately performed. For example, it would be possible to combine or separate some of the steps shown in the various flow diagrams. It would also be possible to just perform a subset of the steps shown in the various flow diagrams. For another example, it is possible to change the boundaries of some of the block diagrams and flow diagrams. It would also be possible, and within the scope of the embodiments described herein, to change the order of some of the steps in the various flow diagram.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments without departing from its scope. While the dimensions, types of materials and coatings described herein are intended to define the parameters of the embodiments of the present technology, they are by no means limiting and are exemplary embodiments. Many other embodiments will be apparent to those of skill in the art upon reviewing the above description. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects. The present invention is set out in the claims that follow.

**Claims**

1. An implantable medical device (102a, 102b, 104, 106), comprising:

conductive communication circuitry (116, 452) configured to transmit conductive communication signals to a plurality of additional implantable medical devices (102a, 102b, 104, 106); and a controller (112, 412) coupled to the conductive communication circuitry (116, 452) and configured to:

generate a first series of bits that are to be communicated to a first of the plurality of additional implantable medical devices (102a, 102b, 104, 106), **characterized in that** the controller (112, 412) is further configured to:

generate a second series of bits that are to be communicated to a second of the plurality of additional implantable medical devices (102a, 102b, 104, 106);
encode the first series of bits and the second series of bits into a series of composite bits, each composite bit of the series of composite bits comprising a first time slot and a second time slot, the first time slot of the composite bit including a respective bit of the first series of bits, and the second time slot of the composite bit including a respective bit of the second series of bits,
wherein, to encode the first series of bits and the second series of bits, the controller (112, 412) is configured to encode each bit of the second series of bits as either a first pseudo noise pulse sequence corresponding to a first bit value of the second series, or a second pseudo noise pulse sequence corresponding to a second bit value of the second series, wherein the first pseudo noise pulse sequence and the second pseudo noise pulse sequence are orthogonal to one another; and
control the conductive communication circuitry (116, 452) to transmit the series of composite bits using conductive communication.

2. The implantable medical device of claim 1, wherein, to encode the first series of bits, the controller (112, 412) is further configured to:
encode each bit of the first series of bits as either a flat waveform corresponding to a first bit value of the first series, or a series of pulses corresponding to a second bit value of the first series of bits.

3. The implantable medical device of any one of claims 1 or 2, wherein the controller (112, 412) is further configured to:

maintain a lookup table of a correspondence between bit values of the second series of bits and the first and second pseudo noise pulse sequences; and
wherein the controller (112, 412) is configured to encode the first series of bits and the second series of bits using the lookup table to translate each bit of second series to the corresponding pseudo noise pulse sequence.

4. The implantable medical device of any one of claims 1 through 3, wherein the series of composite bits is a specified number of composite bits.

5. The implantable medical device of any one of claims 1 through 4, wherein to transmit the series of composite bits the controller (112, 412) is configured to control the conductive communication circuitry (116, 452) to:

transmit a wake up pulse; and
subsequent to the wake up pulse being transmitted, transmit the series of composite bits.

6. The implantable medical device of claim 5, wherein to transmit the series of composite bits the controller (112, 452) is configured to control the conductive communication circuitry (116, 452) to:
wait a specified period of time subsequent to the wake up pulse being transmitted and prior to the series of composite bits being transmitted.

7. The implantable medical device of any one of claims 1 through 6, wherein the series of composite bits comprises a plurality of frames, each of the frames comprising a plurality of the composite bits.

8. A system (100), comprising:

a first implantable medical device, IMD1, which is the implantable medical device (102a, 102b, 104, 106) of any one of claims 1 through 7;
a second implantable medical device, IMD2, which is the first of the plurality of additional implantable medical devices (102a, 102b, 104, 106); and
a third implantable medical device, IMD3, which is the second of the plurality of additional implantable medical devices (102a, 102b, 104, 106).

9. The system of claim 8, wherein the IMD3 is configured to:

receive the series of composite bits; and
decode the received series of composite bits to extract the second series of bits.

10. The system of claim 9, wherein to decode the received series of composite bits and to extract the second series of bits the IMD3 is configured to:

multiply each of the received composite bits by the first pseudo noise pulse sequence;
integrate each of the received composite bits of the second time slot multiplied by the first pseudo noise pulse sequence over a duration;
multiply each of the received composite bits by the second pseudo noise pulse sequence;
integrate each of the received composite bits multiplied by the second pseudo noise pulse sequence over the duration of the second time slot;
map each of the integrated received composite bits multiplied by the first pseudo noise pulse sequence and each of the integrated received composite bits multiplied by the second pseudo noise pulse sequence to a respective in-phase channel value and a respective quadrature channel value; and
determine, from a combination of the in-phase channel values and the quadrature channel values, the value of each of the bits of the second series of bits.

11. The system of claim 10, wherein the IMD3 is further configured to:
determine, from the combination of the in-phase channel values, the quadrature channel values, and the values of the bits of the second series of bits, that data content of the second series of bits as received and decoded is corrupted.

12. The system of any one of claims 8 through 11, wherein to encode the first series of bits and the second series of bits the controller (112, 412) of the IMD1 is configured to:
encode each bit of the first series of bits as either a flat waveform corresponding to the first bit value of the first series, or a series of pulses corresponding to the second bit value of the first series of bits.

13. The system of any one of claims 8 through 11, wherein to encode the first series of bits the controller (112, 412) of the IMD1 is configured to:
encode each bit of the first series of bits as either a third pseudo noise pulse sequence corresponding to a first bit value of the first series, or a fourth pseudo noise pulse sequence corresponding to a second bit value of the first series, wherein the first pseudo noise pulse sequence, the second pseudo noise pulse sequence, the third pseudo noise pulse sequence, and the fourth pseudo noise pulse sequence are mutually orthogonal.

14. The system of any one of claims 8 through 13, wherein:
the IMD3 is configured to transmit a signal intended for reception by the IMD1 using conductive communication, the signal comprising a further series of composite bits each comprising a first time slot including waveform corresponding to a data bit value and a second time slot not containing data;
the IMD1 is further configured to receive the signal intended for reception by the IMD1, and for each bit of the further series of composite bits, determine the data value of the first time slot during the second time slot.

15. The system of any one of claims 8 through 14, wherein:
the IMD1 comprises a first leadless cardiac pacemaker, LCP1 (102a, 102b);
the IMD2 comprises a second leadless cardiac pacemaker, LCP2 (102a, 102b); and
the IMD3 comprises a non-vascular implantable cardioverter defibrillator, NV-ICD (106).

**Patentansprüche**

1. Implantierbare medizinische Vorrichtung (102a, 102b, 104, 106), umfassend:

eine leitende Kommunikationsschaltung (116, 452), die dazu konfiguriert ist, leitende Kommunikationssignale an eine Vielzahl von zusätzlichen implantierbaren medizinischen Vorrichtungen (102a, 102b, 104, 106) zu übertragen; und
eine Steuerung (112, 412), die an die leitende Kommunikationsschaltung (116, 452) gekoppelt und zu Folgendem konfiguriert ist:
Erzeugen einer ersten Reihe von Bits, die an eine erste aus der Vielzahl von zusätzlichen implantierbaren medizinischen Vorrichtungen (102a, 102b, 104, 106) zu kommunizieren ist, **dadurch gekennzeichnet, dass** die Steuerung (112, 412) ferner zu Folgendem konfiguriert ist:

Erzeugen einer zweiten Reihe von Bits, die an eine zweite aus der Vielzahl von zusätzlichen implantierbaren medizinischen Vorrichtungen (102a, 102b, 104, 106) zu kommunizieren ist;
Codieren der ersten Reihe von Bits und der zweiten Reihe von Bits in eine Reihe von zusammengesetzten Bits, wobei jedes zusammengesetzte Bit der Reihe von zusammengesetzten Bits einen ersten Zeitschlitz und einen zweiten Zeitschlitz umfasst, wobei der erste Zeitschlitz des zusammengesetzten Bits ein jeweiliges Bit der ersten Reihe von Bits beinhaltet und der zweite Zeitschlitz des zusammengesetzten Bits ein jeweiliges Bit der zweiten Reihe von Bits beinhaltet,
wobei, um die erste Reihe von Bits und die zweite Reihe von Bits zu codieren, die Steuerung (112, 412) dazu konfiguriert ist, jedes Bit der zweite n Reihe von Bits als entweder eine erste Pseudorauschimpulssequenz entsprechend einem ersten Bitwert der zweiten Reihe oder eine zweite Pseudorauschimpulssequenz entsprechend einem zweiten Bitwert der zweiten Reihe zu codieren, wobei die erste Pseudorauschimpulssequenz und die zweite Pseudorauschimpulssequenz orthogonal zueinander sind; und
Steuern der leitenden Kommunikationsschaltung (116, 452), um die Reihe von zusammengesetzten Bits unter Verwendung von leitender Kommunikation zu übertragen.

2. Implantierbare medizinische Vorrichtung nach Anspruch 1, wobei, um die erste Reihe von Bits zu codieren, die Steuerung (112, 412) ferner zu Folgendem konfiguriert ist:
Codieren jedes Bits der ersten Reihe von Bits als entweder eine flache Wellenform entsprechend einem ersten Bitwert der ersten Reihe oder eine Reihe von Impulsen entsprechend einem zweiten Bitwert der ersten Reihe von Bits.

3. Implantierbare medizinische Vorrichtung nach einem der Ansprüche 1 oder 2, wobei die Steuerung (112, 412) ferner zu Folgendem konfiguriert ist:

Beibehalten einer Nachschlagetabelle einer Entsprechung zwischen Bitwerten der zweiten Reihe von Bits und der ersten und der zweiten Pseudorauschimpulssequenz; und
wobei die Steuerung (112, 412) dazu konfiguriert ist, die erste Reihe von Bits und die zweite Reihe von Bits unter Verwendung der Nachschlagetabelle zu codieren, um jedes Bit der zweiten Reihe zu der entsprechenden Pseudorauschimpulssequenz zu übersetzen.

4. Implantierbare medizinische Vorrichtung nach einem der Ansprüche 1 bis 3, wobei die Reihe von zusammengesetzten Bits eine spezifizierte Anzahl an zusammengesetzten Bits ist.

5. Implantierbare medizinische Vorrichtung nach einem der Ansprüche 1 bis 4, wobei, um die Reihe von zusammengesetzten Bits zu übertragen, die Steuerung (112, 412) dazu konfiguriert ist, die leitende Kommunikationsschaltung (116, 452) zu Folgendem zu steuern:

Übertragen eines Weckimpulses; und
nachdem der Weckimpuls übertragen ist, Übertragen der Reihe von zusammengesetzten Bits.

6. Implantierbare medizinische Vorrichtung nach Anspruch 5, wobei, um die Reihe von zusammengesetzten Bits zu übertragen, die Steuerung (112, 452) dazu konfiguriert ist, die leitende Kommunikationsschaltung (116, 452) zu Folgendem zu steuern:
Warten eines spezifizierten Zeitraums, nachdem der Weckimpuls übertragen ist und bevor die Reihe von zusammengesetzten Bits übertragen ist.

7. Implantierbare medizinische Vorrichtung nach einem der Ansprüche 1 bis 6, wobei die Reihe von zusammengesetzten Bits eine Vielzahl von Frames umfasst, wobei jeder der Frames eine Vielzahl der zusammengesetzten Bits umfasst.

8. System (100), umfassend:

eine erste implantierbare medizinische Vorrichtung, IMD1, welche die implantierbare medizinische Vorrichtung (102a, 102b, 104, 106) nach einem der Ansprüche 1 bis 7 ist;
eine zweite implantierbare medizinische Vorrichtung, IMD2, welche die erste aus der Vielzahl von zusätzlichen implantierbaren medizinischen Vorrichtungen (102a, 102b, 104, 106) ist; und
eine dritte implantierbare medizinische Vorrichtung, IMD3, welche die zweite aus der Vielzahl von zusätzlichen implantierbaren medizinischen Vorrichtungen (102a, 102b, 104, 106) ist.

9. System nach Anspruch 8, wobei die IMD3 zu Folgendem konfiguriert ist:

Empfangen der Reihe von zusammengesetzten Bits; und
Decodieren der empfangenen Reihe von zusammengesetzten Bits, um die zweite Reihe von Bits zu extrahieren.

10. System nach Anspruch 9, wobei, um die empfangene Reihe von zusammengesetzten Bits zu decodieren und um die zweite Reihe von Bits zu extrahieren, die IMD3 zu Folgendem konfiguriert ist:

Multiplizieren von jedem der empfangenen zusammengesetzten Bits mit der ersten Pseudorauschimpulssequenz;
Integrieren von jedem der empfangenen zusammengesetzten Bits des zweiten Zeitschlitzes multipliziert mit der ersten Pseudorauschimpulssequenz über eine Dauer;
Multiplizieren von jedem der empfangenen zusammengesetzten Bits mit der zweiten Pseudorauschimpulssequenz;

Integrieren von jedem der empfangenen zusammengesetzten Bits multipliziert mit der zweiten Pseudorauschimpulssequenz über die Dauer des zweiten Zeitschlitzes;

Zuordnen von jedem der integrierten empfangenen zusammengesetzten Bits multipliziert mit der ersten Pseudorauschimpulssequenz und jedem von den integrierten empfangenen zusammengesetzten Bits multipliziert mit der zweiten Pseudorauschimpulssequenz zu einem jeweiligen gleichphasigen Kanalwert und einem jeweiligen Quadraturkanalwert; und

Bestimmen, aus einer Kombination der gleichphasigen Kanalwerte und der Quadraturkanalwerte, des Wertes von jedem der Bits der zweiten Reihe von Bits.

11. System nach Anspruch 10, wobei die IMD3 ferner zu Folgendem konfiguriert ist:

Bestimmen, aus der Kombination der gleichphasigen Kanalwerte, der Quadraturkanalwerte und der Werte der Bits der zweiten Reihe von Bits, dass Dateninhalt der zweiten Reihe von Bits wie empfangen und decodiert korrupt ist.

12. System nach einem der Ansprüche 8 bis 11, wobei, um die erste Reihe von Bits und die zweite Reihe von Bits zu codieren, die Steuerung (112, 412) der IMD1 zu Folgendem konfiguriert ist:

Codieren jedes Bits der ersten Reihe von Bits als entweder eine flache Wellenform entsprechend dem ersten Bitwert der ersten Reihe oder eine Reihe von Impulsen entsprechend dem zweiten Bitwert der ersten Reihe von Bits.

13. System nach einem der Ansprüche 8 bis 11, wobei, um die erste Reihe von Bits zu codieren, die Steuerung (112, 412) der IMD1 zu Folgendem konfiguriert ist:

Codieren jedes Bits der ersten Reihe von Bits als entweder eine dritte Pseudorauschimpulssequenz entsprechend einem ersten Bitwert der ersten Reihe oder eine vierte Pseudorauschimpulssequenz entsprechend einem zweiten Bitwert der ersten Reihe, wobei die erste Pseudorauschimpulssequenz, die zweite Pseudorauschimpulssequenz, die dritte Pseudorauschimpulssequenz und die vierte Pseudorauschimpulssequenz zueinander orthogonal sind.

14. System nach einem der Ansprüche 8 bis 13, wobei:

die IMD3 dazu konfiguriert ist, ein Signal, das zum Empfang durch die IMD1 vorgesehen ist, unter Verwendung von leitender Kommunikation zu übertragen, wobei das Signal eine weitere Reihe von zusammengesetzten Bits umfasst, die jeweils einen ersten Zeitschlitz, der Wellenform entsprechend einem Datenbitwert beinhaltet, und einen zweiten Zeitschlitz, der keine Daten enthält, umfassen;

die IMD1 ferner dazu konfiguriert ist, das Signal zu empfangen, das zum Empfang durch die IMD1 vorgesehen ist, und für jedes Bit der weiteren Reihe von zusammengesetzten Bits den Datenwert des ersten Zeitschlitzes während des zweiten Zeitschlitzes zu bestimmen.

15. System nach einem der Ansprüche 8 bis 14, wobei:

die IMD1 einen ersten leitungslosen Herzschrittmacher, LCP1, (102a, 102b) umfasst;

die IMD2 einen zweiten leitungslosen Herzschrittmacher, LCP2 (102a, 102b), umfasst; und

die IMD3 einen nichtvaskularen implantierbaren Cardioverter-Defibrillator, NV-ICD (106) umfasst.

## Revendications

1. Dispositif médical implantable (102a, 102b, 104, 106), comprenant :

un circuit de communication conductrice (116, 452) configuré pour transmettre des signaux de communication conductrice à une pluralité de dispositifs médicaux implantables supplémentaires (102a, 102b, 104, 106) ; et

un contrôleur (112, 412) couplé au circuit de communication conductrice (116, 452) et configuré pour :

générer une première série de bits qui doivent être communiqués à un premier de la pluralité de dispositifs médicaux implantables supplémentaires (102a, 102b, 104, 106), **caractérisé en ce que** le contrôleur (112, 412) est en outre configuré pour :

générer une seconde série de bits qui doivent être communiqués à un deuxième de la pluralité de dispositifs médicaux implantables supplémentaires (102a, 102b, 104, 106) ;

coder la première série de bits et la seconde série de bits en une série de bits composites, chaque bit composite de la série de bits composites comprenant un premier intervalle de temps et un second intervalle de temps, le premier intervalle de temps du bit composite incluant un bit respectif de la première série de bits, et le second intervalle de temps du bit composite incluant un bit respectif de la seconde série de bits, dans lequel, pour coder la première série de bits et la seconde série de bits, le contrôleur (112, 412) est configuré pour coder chaque bit de la seconde série de bits sous la forme soit d'une première séquence d'impulsions de pseudo-bruit correspondant à une première valeur de bit de la seconde série, soit d'une deuxième séquence d'impulsions de pseudo-bruit correspondant à une seconde valeur de bit de la seconde série, dans lequel la première séquence d'impulsions de pseudo-bruit et la deuxième séquence d'impulsions de pseudo-bruit sont orthogonales l'une par rapport à l'autre dans laquelle ; et

commander le circuit de communication conductrice (116, 452) pour transmettre la série de bits composites en utilisant une communication conductrice.

2. Dispositif médical implantable selon la revendication 1, dans lequel, pour coder la première série de bits, le contrôleur (112, 412) est en outre configuré pour : coder chaque bit de la première série de bits sous la forme soit d'une forme d'onde plate correspondant à une première valeur de bit de la première série, soit d'une série d'impulsions correspondant à une seconde valeur de bit de la première série de bits.

3. Dispositif médical implantable selon l'une quelconque des revendications 1 ou 2, dans lequel le contrôleur (112, 412) est en outre configuré pour :

   maintenir une table de correspondance d'une correspondance entre des valeurs de bit de la seconde série de bits et les première et deuxième séquences d'impulsions de pseudo-bruit ; et

   dans lequel le contrôleur (112, 412) est configuré pour coder la première série de bits et la seconde série de bits en utilisant la table de correspondance pour traduire chaque bit de la seconde série en la séquence d'impulsions de pseudo-bruit correspondante.

4. Dispositif médical implantable selon l'une quelconque des revendications 1 à 3, dans lequel la série de bits composites est un nombre spécifié de bits composites.

5. Dispositif médical implantable selon l'une quelconque des revendications 1 à 4, dans lequel pour transmettre la série de bits composites, le contrôleur (112, 412) est configuré pour commander le circuit de communication conductrice (116, 452) pour :

   transmettre une impulsion de réveil ; et
   à la suite de la transmission de l'impulsion de réveil, transmettre la série de bits composites.

6. Dispositif médical implantable selon la revendication 5, dans lequel pour transmettre la série de bits composites, le contrôleur (112, 452) est configuré pour commander le circuit de communication conductrice (116, 452) pour :
   attendre une période de temps spécifiée à la suite de la transmission de l'impulsion de réveil et avant la transmission de la série de bits composites.

7. Dispositif médical implantable selon l'une quelconque des revendications 1 à 6, dans lequel la série de bits composites comprend une pluralité de trames, chacune des trames comprenant une pluralité des bits composites.

8. Système (100), comprenant :

   un premier dispositif médical implantable, IMD1, qui est le dispositif médical implantable (102a, 102b, 104, 106) selon l'une quelconque des revendications 1 à 7 ;
   un deuxième dispositif médical implantable, IMD2, qui est le premier de la pluralité de dispositifs médicaux implantables supplémentaires (102a, 102b, 104, 106) ; et
   un troisième dispositif médical implantable, IMD3, qui est le deuxième de la pluralité de dispositifs médicaux implantables supplémentaires (102a, 102b, 104, 106).

9. Système selon la revendication 8, dans lequel l'IMD3 est configuré pour :

   recevoir la série de bits composites ; et
   décoder la série de bits composites reçue pour extraire la seconde série de bits.

10. Système selon la revendication 9, dans lequel pour décoder la série de bits composites reçue et pour extraire la seconde série de bits, l'IMD3 est configuré pour :

    multiplier chacun des bits composites reçus par la première séquence d'impulsions de pseudo-bruit ;
    intégrer, sur une durée, chacun des bits composites reçus du second intervalle de temps multipliés par la première séquence d'impulsions de pseudo-bruit ;
    multiplier chacun des bits composites reçus par la deuxième séquence d'impulsions de pseudo-bruit ;
    intégrer chacun des bits composites reçus multipliés par la deuxième séquence d'impulsions de pseudo-bruit sur la durée du second intervalle de temps ;
    mettre en correspondance chacun des bits composites reçus intégrés multipliés par la première séquence d'impulsions de pseudo-bruit et chacun des bits composites reçus intégrés multipliés par la deuxième séquence d'impulsions de pseudo-bruit avec une valeur de canal en phase respective et une valeur de canal en quadrature respective ; et

déterminer, à partir d'une combinaison des valeurs de canal en phase et des valeurs de canal en quadrature, la valeur de chacun des bits de la seconde série de bits.

11. Système selon la revendication 10, dans lequel l'IMD3 est en outre configuré pour :

déterminer, à partir de la combinaison des valeurs de canal en phase, des valeurs de canal en quadrature, et des valeurs des bits de la seconde série de bits, qu'un contenu de données de la seconde série de bits tel que reçu et décodé est corrompu.

12. Système selon l'une quelconque des revendications 8 à 11, dans lequel pour coder la première série de bits et la seconde série de bits, le contrôleur (112, 412) de l'IMD1 est configuré pour :

coder chaque bit de la première série de bits sous la forme soit d'une forme d'onde plate correspondant à la première valeur de bit de la première série, soit d'une série d'impulsions correspondant à la seconde valeur de bit de la première série de bits.

13. Système selon l'une quelconque des revendications 8 à 11, dans lequel pour coder la première série de bits, le contrôleur (112, 412) de l'IMD1 est configuré pour :

coder chaque bit de la première série de bits sous la forme soit d'une troisième séquence d'impulsions de pseudo-bruit correspondant à une première valeur de bit de la première série, soit d'une quatrième séquence d'impulsions de pseudo-bruit correspondant à une seconde valeur de bit de la première série, dans lequel la première séquence d'impulsions de pseudo-bruit, la deuxième séquence d'impulsions de pseudo-bruit, la troisième séquence d'impulsions de pseudo-bruit, et la quatrième séquence d'impulsions de pseudo-bruit sont mutuellement orthogonales.

14. Système selon l'une quelconque des revendications 8 à 13, dans lequel :

l'IMD3 est configuré pour transmettre un signal destiné à être reçu par l'IMD1 en utilisant une communication conductrice, le signal comprenant une autre série de bits composites comprenant chacun un premier intervalle de temps incluant une forme d'onde correspondant à une valeur de bit de données et un second intervalle de temps ne contenant pas de données ;

l'IMD1 est en outre configuré pour recevoir le signal destiné à être reçu par l'IMD1, et pour chaque bit de l'autre série de bits composites, déterminer la valeur de données du premier intervalle de temps pendant le second intervalle de temps.

15. Système selon l'une quelconque des revendications 8 à 14, dans lequel :

l'IMD1 comprend un premier d'un stimulateur cardiaque sans sonde, LCP1 (102a, 102b) ; l'IMD2 comprend un deuxième stimulateur cardiaque sans sonde, LCP2 (102a, 102b) ; et l'IMD3 comprend un défibrillateur cardioverteur implantable non vasculaire, NV-ICD (106).

**FIG. 1**

**FIG. 2**

**FIG. 3**

**FIG. 4**

One (composite) bit time duration of LCP transmitting signal

Square wave pulses

Time slot 1

Time slot 2

Orthogonal Pseudo-Noise (PN) pulse sequences $PN_0$ or $PN_1$

LCP Transmitting Signal

## FIG. 5

One bit time duration of S-ICD transmitting signal

Square wave pulses

Time slot 1

Time slot 2

S-ICD Transmitting Signal

## FIG. 6

EP 4 601 737 B1

PN pulse encoded data payload frames

| Fixed silent time period | Frame 1 | Frame 2 | $\cdots$ | Frame N |

↑ Wake up pulse

| cb 1 | cb 2 | ... | cb m |

## FIG. 7

**bit 1 waveform**

Time (sec) x $10^{-5}$

**bit 0 waveform**

Time (sec) x $10^{-5}$

## FIG. 8

EP 4 601 737 B1

Transmitting bits:
[0, 1, 1, 1]
901

"0"=flat
"1"=pulse
903

Transmitting pulses:
[f, p, p, p]
905

Composite bits:
[f/PN0, p/PN1, p/PN0, p/PN1]
921

Transmitting bits:
[0, 1, 0, 1]
911

LUT:
"0"=$PN_0$
"1"=$PN_1$
913

Transmitting pulses:
[PN0, PN1, PN0, PN1]
915

**FIG. 9**

Generate a first series of bits that are to be communicated from IMD1 to IMD2 — 1001

↓

Encode first series of bits by IMD1 — 1003

↓

Generate a second series of bits that are to be communicated from IMD1 to IMD3 — 1005

↓

Encode second series of bits by IMD1 as orthogonal pseudo noise pulse sequences by IMD1 — 1007

↓

Combine first encoded series of bits and second encoded series of bits into composite bits by IMD1 — 1009

↓

Format composite bits into frames by IMD1 — 1011

↓

Transmit by IMD1 of frames by conductive communication — 1013

*FIG. 10*

**FIG. 11**

I channel

Q channel

I Q channel mapper (e.g., I channel = log(X/Y), Q channel = log(Y/X)) — 1125

X: integrator 1 output

Integrator 1 — 1105

Multiplier — 1103

PN$_1$ signal generator — 1101

Signal Splitter — 1123

Buffer storing received signal representing one bit of message — 1121

Y: integrator 0 output

Integrator 0 — 1115

Multiplier — 1113

PN$_0$ signal generator — 1111

*FIG. 12*

PN pulse encoded data
payload frames

| | Fixed silent time period | Public Frame(s): Encoded with the public {PN_0, PN_1} pair | Public Frame(s): Encoded with the private {PN_0, PN_1} pair (pair ID is from public frame) |
| --- | --- | --- | --- |
| | | Contains:<br>1. Receiving device ID<br>2. Private {PN_0, PN_1} pair ID (optional) | Contains:<br>Private session data |

Wake up pulse

## FIG. 13

Transmitting Device

Start — 1401

Transmit wake up pulse — 1403

Wait for Fixed Silent Time Period — 1405

Encode Public Frame with the public {PN$_0$, PN$_1$} pair — 1407

Transmit the Public Frame — 1409

Encode Private Frame with the private {PN$_0$, PN$_1$} pair — 1411

Transmit the Private Frame — 1413

End — 1415

Receiving Device

Start — 1431

Wake up pulse → Receive wake up pulse — 1433

Wait for Fixed Silent Time Period — 1435

Public Frame → Receive and decode the Public Frame with the public {PN$_0$, PN$_1$} pair to obtain device ID — 1437

Device ID matched? — 1439

No

Yes

Switch to private {PN$_0$, PN$_1$} pair — 1441

Private Frame → Receive and decode the Private Frame with the private {PN$_0$, PN$_1$} pair — 1443

End — 1445

FIG. 14

**FIG. 15**

**FIG. 16**

PN pulse encoded data payload frames

Fixed silent time period

Encoded with the $\{PN_0, PN_1\}$ pair of the receiving device

Contains:
Private session data

Wake up pulse

**FIG. 17**

EP 4 601 737 B1

Transmitting Device

Start 1801

Transmit wake up pulse 1803 —— Wake up pulse ——>

Wait for Fixed Silent Time Period 1805

Look up the {$PN_0$, $PN_1$} pair of the receiving device 1807

Encode the data frame(s) with the {$PN_0$, $PN_1$} pair of the receiving device 1809

Transmit the encoded private frame(s) 1811 —— Data Frame ——>

End 1813

Receiving Device

Start 1831

Receive wake up pulse 1833

Wait for Fixed Silent Time Period 1835

Receive and decode the private data frame(s) with the private {$PN_0$, $PN_1$} pair 1841

End 1843

**FIG. 18**

Private Frame encoded with {PN$_0$, PN$_1$} for device 2

Private Frame encoded with {PN$_0$, PN$_1$} for device 1

Private Frame encoded with {PN$_0$, PN$_1$} for device 1

Private Frame encoded with {PN$_0$, PN$_1$} for device 3

Device 1
1901

Device 2
1902

Device 3
1903

Private Frame encoded with {PN$_0$, PN$_1$} for device 3

Private Frame encoded with {PN$_0$, PN$_1$} for device 2

**FIG. 19**

Master Device 1
2001

Slave Device 1
2011

Slave Device 2
2012

Private Frame encoded with {PN$_0$, PN$_1$} for slave device 1

Private Frame encoded with {PN$_0$, PN$_1$} for master device

Private Frame encoded with {PN$_0$, PN$_1$} for slave device 2

Private Frame encoded with {PN$_0$, PN$_1$} for master device

**FIG. 20**

PN Pulse Encoded Waveform with (No Noise)

Time (seconds)

Decoded Constellation Diagram on I-Q Channels

Decoded "Bit 0"
(22 "Bit 0"s in total)

Null Region

Decoded "Bit 1"
(10 "Bit 1"s in total)

Q Channel

I Channel

*FIG. 21*

EP 4 601 737 B1

PN Pulse Encoded Waveform with added Gaussian Noise (SNR≈0dB)

Time (seconds)

x10$^{-2}$

Decoded Constellation Diagram on I-Q Channels

Q Channel

Decoded "Bit 0"
(22 "Bit 0"s in total)

Null Region

Decoded "Bit 1"
(10 "Bit 1"s in total)

I Channel

FIG. 22

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2022370810 A1
- US 20220370810 A
- US 24138919 A
- US 20200215536 A1